# EUROPEAN PATENT APPLICATION

(11) **EP 1 016 723 A1**
(43) Date of publication of application: **05.07.2000**
(21) Application number: 99310191.4
(22) Date of filing: 17.12.1999
(51) Int. Cl.: C12N 15/57, C12N 9/48, C12N 15/16, C07K 14/67, C07K 14/635, C07K 14/605, C12N 15/62, C07K 19/00

(54) **Recombinant synthesis of beta-Lipotropin and other peptides**

(30) Priority: 21.12.1998 US 113058 P
(71) Applicant: ELI LILLY AND COMPANY, Indianapolis, Indiana 46285 (US)
(72) Inventor: Hale, John Edward, Fishers, Indiana 46038 (US); Hershberger, Charles Lee, Greenfield, Indiana 46140 (US); Larson, Jeffrey Lynn, Indianapolis, Indiana 46260 (US); Menke, Michael Andrew, Indianapolis, Indiana 46219 (US)
(74) Representative: Denholm, Anna Marie

(57) **Abstract**

The invention provides isolated nucleic acids, vectors, transformed host cells, fusion proteins, and recombinant methods for producing proteins using the propeptide sequence of procarboxypeptidase B.

### Annex to the description

SEQUENCE LISTING

<110> Hershberger, Charles L.
      Hale, John E.
      Menke, Michael A.
      Larson, Jeffrey L.

<120> Recombinant Synthesis of Beta-lipotropin and Other
      Peptides

<130> X-12579

<140>
<141>

<160> 53

<170> PatentIn Ver. 2.0

<210> 1
<211> 279
<212> DNA
<213> Porcine

<220>
<221> CDS
<222> (1)..(279)

<400> 1

cac cac tcc ggg gag cat ttc gaa ggg gag aag gtg ttc cgt gtc aat    48

His His Ser Gly Glu His Phe Glu Gly Glu Lys Val Phe Arg Val Asn

1               5                   10                  15

gtt gaa gat gaa aat gac atc agc tta ctc cat gag ttg gcc agc acc    96

Val Glu Asp Glu Asn Asp Ile Ser Leu Leu His Glu Leu Ala Ser Thr

            20                  25                  30

agg cag att gac ttc tgg aaa cca gat tct gtc aca caa atc aaa cct    144

Arg Gln Ile Asp Phe Trp Lys Pro Asp Ser Val Thr Gln Ile Lys Pro

        35                  40                  45

cac agt aca gtt gac ttc cgt gtg aaa gca gaa gat att ttg gct gtg    192

His Ser Thr Val Asp Phe Arg Val Lys Ala Glu Asp Ile Leu Ala Val

    50                  55                  60

gaa gac ttt ctg gag cag aat gaa cta caa tat gag gta ctc ata aac    240

Glu Asp Phe Leu Glu Gln Asn Glu Leu Gln Tyr Glu Val Leu Ile Asn

65                  70                  75                  80

aac ctg aga tct gtg ctc gag gct cag ttt gac agc aga                279

Asn Leu Arg Ser Val Leu Glu Ala Gln Phe Asp Ser Arg

                85                  90

<210> 2

<211> 93

<212> PRT

<213> Porcine

<400> 2

His His Ser Gly Glu His Phe Glu Gly Glu Lys Val Phe Arg Val Asn

1               5                   10                  15

Val Glu Asp Glu Asn Asp Ile Ser Leu Leu His Glu Leu Ala Ser Thr

            20                  25                  30

Arg Gln Ile Asp Phe Trp Lys Pro Asp Ser Val Thr Gln Ile Lys Pro

        35                  40                  45

His Ser Thr Val Asp Phe Arg Val Lys Ala Glu Asp Ile Leu Ala Val

    50                  55                  60

Glu Asp Phe Leu Glu Gln Asn Glu Leu Gln Tyr Glu Val Leu Ile Asn

65                  70                  75                  80

Asn Leu Arg Ser Val Leu Glu Ala Gln Phe Asp Ser Arg

                85                  90

<210> 3

<211> 807

<212> DNA

<213> Artificial Sequence

<220>

<223> Description of Artificial Sequence:aMF-PCpB-BLT
      fusion

<220>
<221> CDS
<222> (1)..(801)

<400> 3
atg aga ttt cct tca att ttt act gca gtt tta ttc gca gca tcc tcc    48
gca tta gct gct cca gtc aac act aca aca gaa gat gaa acg gca caa    96
att ccg gct gaa gct gtc atc ggt tac tca gat tta gaa ggg gat ttc   144
gat gtt gct gtt ttg cca ttt tcc aac agc aca aat aac ggg tta ttg   192
ttt ata aat act act att gcc agc att gct gct aaa gaa gaa ggg gta   240
tct ctc gag aaa aga cac tgg cac cac cac cac tcc ggg gag cat ttc 288
gaa ggg gag aag gtg ttc cgt gtc aat gtt gaa gat gaa aat gac atc 336
agc tta ctc cat gag ttg gcc agc acc agg cag att gac ttc tgg aaa 384
cca gat tct gtc aca caa atc aaa cct cac agt aca gtt gac ttc cgt 432
gtg aaa gca gaa gat att ttg gct gtg gaa gac ttt ctg gag cag aat 480
gaa cta caa tat gag gta ctc ata aac aac ctg aga tct gtg ctc gag 528
aaa aga gaa ctg acc ggt cag cgt ctg cgc gaa ggt gac ggt ccg gac 576
ggt ccg gct gac gac ggt gct ggt gct cag gca gat ctg gaa cac tcc 624
ctg ctg gtt gct gca gag aag aaa gac gaa ggt ccg tac cgt atg gaa 672
cac ttc cgt tgg ggt tcc ccg ccg aaa gac aag cgt tac ggt ggt ttc 720
atg acc tcc gag aaa tcc cag acc ccg ctg gtt act ctg ttc aag aac 768
gct atc atc aag aac gcg tac aag aaa ggt gaa taatag 807

<210> 4
<211> 267
<212> PRT
<213> Artificial Sequence

<400> 4

<210> 5

<211> 573

<212> DNA

<213> Artificial Sequence

<220>

<223> Description of Artificial Sequence: PCpB-LVPR-BLT
      fusion

<220>
<221> CDS
<222> (4)..(561)

<400> 5
cat atg cat cat agt ggt gaa cat ttc gaa ggg gag aag gtg ttc cgt    48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                 20                  25                  30

agc acc agg cag att gac ttc tgg aaa cca gat tct gtc aca caa atc    144
Ser Thr Arg Gln Ile Asp Phe Trp Lys Pro Asp Ser Val Thr Gln Ile
             35                  40                  45

aaa cct cac agt aca gtt gac ttc cgt gtg aaa gca gaa gat att ttg    192
Lys Pro His Ser Thr Val Asp Phe Arg Val Lys Ala Glu Asp Ile Leu
         50                  55                  60

gct gtg gaa gac ttt ctg gag cag aat gaa cta caa tat gag gta ctc    240
Ala Val Glu Asp Phe Leu Glu Gln Asn Glu Leu Gln Tyr Glu Val Leu

65                  70                  75

ata aac aac ctg aga tct gtg ctc gag gct cag ttt gac agc ctg gta    288
Ile Asn Asn Leu Arg Ser Val Leu Glu Ala Gln Phe Asp Ser Leu Val
80                  85                  90                  95

ccg cgt gaa ctg acc ggt cag cgt ctg cgc gaa ggt gac ggt ccg gac    336
Pro Arg Glu Leu Thr Gly Gln Arg Leu Arg Glu Gly Asp Gly Pro Asp
                100                 105                 110

ggt ccg gct gac gac ggt gct ggt gct cag gca gat ctg gaa cac tcc    384
Gly Pro Ala Asp Asp Gly Ala Gly Ala Gln Ala Asp Leu Glu His Ser
            115                 120                 125

ctg ctg gtt gct gca gag aag aaa gac gaa ggt ccg tac cgt atg gaa    432
Leu Leu Val Ala Ala Glu Lys Lys Asp Glu Gly Pro Tyr Arg Met Glu
        130                 135                 140

cac ttc cgt tgg ggt tcc ccg ccg aaa gac aag cgt tac ggt ggt ttc    480
His Phe Arg Trp Gly Ser Pro Pro Lys Asp Lys Arg Tyr Gly Gly Phe
    145                 150                 155

atg acc tcc gag aaa tcc cag acc ccg ctg gtt act ctg ttc aag aac    528
Met Thr Ser Glu Lys Ser Gln Thr Pro Leu Val Thr Leu Phe Lys Asn
160                 165                 170                 175

gct atc atc aag aac gcg tac aag aaa ggt gaa taatagggat cc          573
Ala Ile Ile Lys Asn Ala Tyr Lys Lys Gly Glu

                180                 185

<210> 6

<211> 186

<212> PRT

<213> Artificial Sequence

<400> 6

Met His His Ser Gly Glu His Phe Glu Gly Glu Lys Val Phe Arg Val

1               5                   10                  15

Asn Val Glu Asp Glu Asn Asp Ile Ser Leu Leu His Glu Leu Ala Ser

            20                  25                  30

Thr Arg Gln Ile Asp Phe Trp Lys Pro Asp Ser Val Thr Gln Ile Lys

        35                  40                  45

Pro His Ser Thr Val Asp Phe Arg Val Lys Ala Glu Asp Ile Leu Ala

    50                  55                  60

Val Glu Asp Phe Leu Glu Gln Asn Glu Leu Gln Tyr Glu Val Leu Ile

65                  70                  75                  80

Asn Asn Leu Arg Ser Val Leu Glu Ala Gln Phe Asp Ser Leu Val Pro

                85                  90                  95

Arg Glu Leu Thr Gly Gln Arg Leu Arg Glu Gly Asp Gly Pro Asp Gly

100                 105                 110

Pro Ala Asp Asp Gly Ala Gly Ala Gln Ala Asp Leu Glu His Ser Leu

        115                 120                 125

Leu Val Ala Ala Glu Lys Lys Asp Glu Gly Pro Tyr Arg Met Glu His

    130                 135                 140

Phe Arg Trp Gly Ser Pro Pro Lys Asp Lys Arg Tyr Gly Gly Phe Met

145                 150                 155                 160

Thr Ser Glu Lys Ser Gln Thr Pro Leu Val Thr Leu Phe Lys Asn Ala

                165                 170                 175

Ile Ile Lys Asn Ala Tyr Lys Lys Gly Glu

            180                 185

<210> 7

<211> 405

<212> DNA

<213> Artificial Sequence

<220>

<223> Description of Artificial Sequence: PCpB-RVR-hPTH

      fusion

<220>

<221> CDS

<222> (4)..(393)

<400> 7

cat atg cat cat agt ggt gaa cat ttc gaa ggg gag aag gtg ttc cgt    48

    Met His His Ser Gly Glu His Phe Glu Gly Glu Lys Val Phe Arg

    1               5                   10                  15

gtc aat gtt gaa gat gaa aat gac atc agc tta ctc cat gag ttg gcc    96

Val Asn Val Glu Asp Glu Asn Asp Ile Ser Leu Leu His Glu Leu Ala

                20                  25                  30

agc acc agg cag att gac ttc tgg aaa cca gat tct gtc aca caa atc    144

Ser Thr Arg Gln Ile Asp Phe Trp Lys Pro Asp Ser Val Thr Gln Ile

            35                  40                  45

aaa cct cac agt aca gtt gac ttc cgt gtg aaa gca gaa gat att ttg    192

Lys Pro His Ser Thr Val Asp Phe Arg Val Lys Ala Glu Asp Ile Leu

        50                  55                  60

gct gtg gaa gac ttt ctg gag cag aat gaa cta caa tat gag gta ctc    240

Ala Val Glu Asp Phe Leu Glu Gln Asn Glu Leu Gln Tyr Glu Val Leu

    65                  70                  75

ata aac aac ctg aga tct gtg ctc gag gct cag ttt gac agc aga gtc    288

Ile Asn Asn Leu Arg Ser Val Leu Glu Ala Gln Phe Asp Ser Arg Val

80                  85                  90                  95

cgt tct gtt tct gaa atc cag ctg atg cat aac ctg ggc aaa cat ctg 336
Arg Ser Val Ser Glu Ile Gln Leu Met His Asn Leu Gly Lys His Leu
                100                 105                 110

aac tct atg gag cgt gta gaa tgg ctg cgt aag aag ctg cag gat gtt 384
Asn Ser Met Glu Arg Val Glu Trp Leu Arg Lys Lys Leu Gln Asp Val
            115                 120                 125

cac aac ttt tgatagggat cc 405
His Asn Phe
        130

<210> 8
<211> 130
<212> PRT
<213> Artificial Sequence

<400> 8
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Arg Val Arg
                85                  90                  95

Ser Val Ser Glu Ile Gln Leu Met His Asn Leu Gly Lys His Leu Asn
            100                 105                 110

Ser Met Glu Arg Val Glu Trp Leu Arg Lys Lys Leu Gln Asp Val His
        115                 120                 125

Asn Phe
    130

<210> 9
<211> 411
<212> DNA
<213> Artificial Sequence

<220>
<223> Description of Artificial Sequence: PCpB-ALY-hPTH
fusion

<220>

<221> CDS

<222> (4)..(399)


<400> 9

cat atg cat cat agt ggt gaa cat ttc gaa ggg gag aag gtg ttc cgt    48

    Met His His Ser Gly Glu His Phe Glu Gly Glu Lys Val Phe Arg

    1               5                   10                  15


gtc aat gtt gaa gat gaa aat gac atc agc tta ctc cat gag ttg gcc    96

Val Asn Val Glu Asp Glu Asn Asp Ile Ser Leu Leu His Glu Leu Ala

                20                  25                  30


agc acc agg cag att gac ttc tgg aaa cca gat tct gtc aca caa atc    144

Ser Thr Arg Gln Ile Asp Phe Trp Lys Pro Asp Ser Val Thr Gln Ile

            35                  40                  45


aaa cct cac agt aca gtt gac ttc cgt gtg aaa gca gaa gat att ttg    192

Lys Pro His Ser Thr Val Asp Phe Arg Val Lys Ala Glu Asp Ile Leu

        50                  55                  60


gct gtg gaa gac ttt ctg gag cag aat gaa cta caa tat gag gta ctc    240

Ala Val Glu Asp Phe Leu Glu Gln Asn Glu Leu Gln Tyr Glu Val Leu

    65                  70                  75


ata aac aac ctg aga tct gtg ctc gag gct cag ttt gac tca cgc gtt    288

Ile Asn Asn Leu Arg Ser Val Leu Glu Ala Gln Phe Asp Ser Arg Val

80                  85                  90                  95

gca ctg tat tct gtt tct gaa atc cag ctg atg cat aac ctg ggc aaa    336
Ala Leu Tyr Ser Val Ser Glu Ile Gln Leu Met His Asn Leu Gly Lys
                100                 105                 110

cat ctg aac tct atg gag cgt gta gaa tgg ctg cgt aag aag ctg cag    384
His Leu Asn Ser Met Glu Arg Val Glu Trp Leu Arg Lys Lys Leu Gln
            115                 120                 125

gat gtt cac aac ttt tgatagggat cc                                  411
Asp Val His Asn Phe
        130

<210> 10
<211> 132
<212> PRT
<213> Artificial Sequence

<400> 10
Met His His Ser Gly Glu His Phe Glu Gly Glu Lys Val Phe Arg Val
  1               5                  10                  15

Asn Val Glu Asp Glu Asn Asp Ile Ser Leu Leu His Glu Leu Ala Ser
                20                  25                  30

Thr Arg Gln Ile Asp Phe Trp Lys Pro Asp Ser Val Thr Gln Ile Lys
            35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Arg Val Ala
                85                  90                  95

Leu Tyr Ser Val Ser Glu Ile Gln Leu Met His Asn Leu Gly Lys His
            100                 105                 110

Leu Asn Ser Met Glu Arg Val Glu Trp Leu Arg Lys Lys Leu Gln Asp
        115                 120                 125

Val His Asn Phe
    130

<210> 11

<211> 408

<212> DNA

<213> Artificial Sequence

<220>

<223> Description of Artificial Sequence: PCpB-IEGR-hPTH
fusion

<220>

<221> CDS

<222> (4)..(396)

<400> 11

cat atg cat cat agt ggt gaa cat ttc gaa ggg gag aag gtg ttc cgt 48
Met His His Ser Gly Glu His Phe Glu Gly Glu Lys Val Phe Arg
1               5                   10                  15

gtc aat gtt gaa gat gaa aat gac atc agc tta ctc cat gag ttg gcc 96
Val Asn Val Glu Asp Glu Asn Asp Ile Ser Leu Leu His Glu Leu Ala
                20                  25                  30

agc acc agg cag att gac ttc tgg aaa cca gat tct gtc aca caa atc 144
Ser Thr Arg Gln Ile Asp Phe Trp Lys Pro Asp Ser Val Thr Gln Ile
            35                  40                  45

aaa cct cac agt aca gtt gac ttc cgt gtg aaa gca gaa gat att ttg 192
Lys Pro His Ser Thr Val Asp Phe Arg Val Lys Ala Glu Asp Ile Leu
        50                  55                  60

gct gtg gaa gac ttt ctg gag cag aat gaa cta caa tat gag gta ctc 240
Ala Val Glu Asp Phe Leu Glu Gln Asn Glu Leu Gln Tyr Glu Val Leu
    65                  70                  75

ata aac aac ctg aga tct gtg ctc gag gct cag ttt gac tcg atc gaa 288
Ile Asn Asn Leu Arg Ser Val Leu Glu Ala Gln Phe Asp Ser Ile Glu

80                  85                  90                  95

ggc cgt tct gtt tct gaa atc cag ctg atg cat aac ctg ggc aaa cat    336

Gly Arg Ser Val Ser Glu Ile Gln Leu Met His Asn Leu Gly Lys His

                100                 105                 110

ctg aac tct atg gag cgt gta gaa tgg ctg cgt aag aag ctg cag gat    384

Leu Asn Ser Met Glu Arg Val Glu Trp Leu Arg Lys Lys Leu Gln Asp

            115                 120                 125

gtt cac aac ttt tgatagggat cc                                      408

Val His Asn Phe

        130

<210> 12

<211> 131

<212> PRT

<213> Artificial Sequence

<400> 12

Met His His Ser Gly Glu His Phe Glu Gly Glu Lys Val Phe Arg Val

  1               5                  10                  15

Asn Val Glu Asp Glu Asn Asp Ile Ser Leu Leu His Glu Leu Ala Ser

                20                  25                  30

Thr Arg Gln Ile Asp Phe Trp Lys Pro Asp Ser Val Thr Gln Ile Lys

        35                  40                  45

Pro His Ser Thr Val Asp Phe Arg Val Lys Ala Glu Asp Ile Leu Ala

    50                  55                  60

Val Glu Asp Phe Leu Glu Gln Asn Glu Leu Gln Tyr Glu Val Leu Ile

65                  70                  75                  80

Asn Asn Leu Arg Ser Val Leu Glu Ala Gln Phe Asp Ser Ile Glu Gly

                85                  90                  95

Arg Ser Val Ser Glu Ile Gln Leu Met His Asn Leu Gly Lys His Leu

            100                 105                 110

Asn Ser Met Glu Arg Val Glu Trp Leu Arg Lys Lys Leu Gln Asp Val

        115                 120                 125

His Asn Phe

    130

<210> 13

<211> 408

<212> DNA

<213> Artificial Sequence

<220>

<223> Description of Artificial Sequence: PCpB-LVPR-hPTH

fusion

<220>
<221> CDS
<222> (4)..(396)

<400> 13
cat atg cat cat agt ggt gaa cat ttc gaa ggg gag aag gtg ttc cgt    48
    Met His His Ser Gly Glu His Phe Glu Gly Glu Lys Val Phe Arg
    1               5                   10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                20                  25                  30

agc acc agg cag att gac ttc tgg aaa cca gat tct gtc aca caa atc    144
Ser Thr Arg Gln Ile Asp Phe Trp Lys Pro Asp Ser Val Thr Gln Ile
            35                  40                  45

aaa cct cac agt aca gtt gac ttc cgt gtg aaa gca gaa gat att ttg    192
Lys Pro His Ser Thr Val Asp Phe Arg Val Lys Ala Glu Asp Ile Leu
        50                  55                  60

gct gtg gaa gac ttt ctg gag cag aat gaa cta caa tat gag gta ctc    240
Ala Val Glu Asp Phe Leu Glu Gln Asn Glu Leu Gln Tyr Glu Val Leu
    65                  70                  75

ata aac aac ctg aga tct gtg ctc gag gct cag ttt gac agc ctg gta    288

Ile Asn Asn Leu Arg Ser Val Leu Glu Ala Gln Phe Asp Ser Leu Val
80                  85                  90                  95

ccg cgt tct gtt tct gaa atc cag ctg atg cat aac ctg ggc aaa cat     336
Pro Arg Ser Val Ser Glu Ile Gln Leu Met His Asn Leu Gly Lys His
                100                 105                 110

ctg aac tct atg gag cgt gta gaa tgg ctg cgt aag aag ctg cag gat     384
Leu Asn Ser Met Glu Arg Val Glu Trp Leu Arg Lys Lys Leu Gln Asp
            115                 120                 125

gtt cac aac ttt tgatagggat cc                                       408
Val His Asn Phe
        130

<210> 14
<211> 131
<212> PRT
<213> Artificial Sequence

<400> 14
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Leu Val Pro
                85                  90                  95

Arg Ser Val Ser Glu Ile Gln Leu Met His Asn Leu Gly Lys His Leu
            100                 105                 110

Asn Ser Met Glu Arg Val Glu Trp Leu Arg Lys Lys Leu Gln Asp Val
        115                 120                 125

His Asn Phe
    130

<210> 15

<211> 417

<212> DNA

<213> Artificial Sequence

<220>

<223> Description of Artificial Sequence: PCpB-VIPR-hPTH
      fusion

<220>
<221> CDS
<222> (4)..(405)

<400> 15
cat atg cat cat agt ggt gaa cat ttc gaa ggg gag aag gtg ttc cgt    48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                 20                  25                  30

agc acc agg cag att gac ttc tgg aaa cca gat tct gtc aca caa atc   144
Ser Thr Arg Gln Ile Asp Phe Trp Lys Pro Asp Ser Val Thr Gln Ile
             35                  40                  45

aaa cct cac agt aca gtt gac ttc cgt gtg aaa gca gaa gat att ttg   192
Lys Pro His Ser Thr Val Asp Phe Arg Val Lys Ala Glu Asp Ile Leu
         50                  55                  60

gct gtg gaa gac ttt ctg gag cag aat gaa cta caa tat gag gta ctc   240
Ala Val Glu Asp Phe Leu Glu Gln Asn Glu Leu Gln Tyr Glu Val Leu
     65                  70                  75

ata aac aac ctg aga tct gtg ctc gag gct cag ttt gac agc aga gtc 288
Ile Asn Asn Leu Arg Ser Val Leu Glu Ala Gln Phe Asp Ser Arg Val
80                  85                  90                  95

cgt gtt atc cca agg tct gtt tct gaa atc cag ctg atg cat aac ctg 336
Arg Val Ile Pro Arg Ser Val Ser Glu Ile Gln Leu Met His Asn Leu
                100                 105                 110

ggc aaa cat ctg aac tct atg gag cgt gta gaa tgg ctg cgt aag aag 384
Gly Lys His Leu Asn Ser Met Glu Arg Val Glu Trp Leu Arg Lys Lys
            115                 120                 125

ctg cag gat gtt cac aac ttt tgatagggat cc 417
Leu Gln Asp Val His Asn Phe
        130

<210> 16
<211> 134
<212> PRT
<213> Artificial Sequence

<400> 16
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Arg Val Arg
                85                  90                  95

Val Ile Pro Arg Ser Val Ser Glu Ile Gln Leu Met His Asn Leu Gly
            100                 105                 110

Lys His Leu Asn Ser Met Glu Arg Val Glu Trp Leu Arg Lys Lys Leu
        115                 120                 125

Gln Asp Val His Asn Phe
    130

<210> 17
<211> 423
<212> DNA
<213> Artificial Sequence

<220>

<223> Description of Artificial Sequence:

      PCpB-DQVDPR-hPTH fusion

<220>

<221> CDS

<222> (4)..(411)

<400> 17

cat atg cat cat agt ggt gaa cat ttc gaa ggg gag aag gtg ttc cgt    48

    Met His His Ser Gly Glu His Phe Glu Gly Glu Lys Val Phe Arg

      1               5                  10                  15

gtc aat gtt gaa gat gaa aat gac atc agc tta ctc cat gag ttg gcc    96

Val Asn Val Glu Asp Glu Asn Asp Ile Ser Leu Leu His Glu Leu Ala

                 20                  25                  30

agc acc agg cag att gac ttc tgg aaa cca gat tct gtc aca caa atc    144

Ser Thr Arg Gln Ile Asp Phe Trp Lys Pro Asp Ser Val Thr Gln Ile

             35                  40                  45

aaa cct cac agt aca gtt gac ttc cgt gtg aaa gca gaa gat att ttg    192

Lys Pro His Ser Thr Val Asp Phe Arg Val Lys Ala Glu Asp Ile Leu

         50                  55                  60

gct gtg gaa gac ttt ctg gag cag aat gaa cta caa tat gag gta ctc    240

Ala Val Glu Asp Phe Leu Glu Gln Asn Glu Leu Gln Tyr Glu Val Leu

     65                  70                  75

ata aac aac ctg aga tct gtg ctc gag gct cag ttt gac agc aga gtc    288
Ile Asn Asn Leu Arg Ser Val Leu Glu Ala Gln Phe Asp Ser Arg Val
 80                  85                  90                  95

cgt gac cag gtc gac ccg cgt tct gtt tct gaa atc cag ctg atg cat    336
Arg Asp Gln Val Asp Pro Arg Ser Val Ser Glu Ile Gln Leu Met His
                100                 105                 110

aac ctg ggc aaa cat ctg aac tct atg gag cgt gta gaa tgg ctg cgt    384
Asn Leu Gly Lys His Leu Asn Ser Met Glu Arg Val Glu Trp Leu Arg
            115                 120                 125

aag aag ctg cag gat gtt cac aac ttt tgatagggat cc                  423
Lys Lys Leu Gln Asp Val His Asn Phe
        130                 135

<210> 18
<211> 136
<212> PRT
<213> Artificial Sequence

<400> 18
Met His His Ser Gly Glu His Phe Glu Gly Glu Lys Val Phe Arg Val
  1               5                  10                  15

Asn Val Glu Asp Glu Asn Asp Ile Ser Leu Leu His Glu Leu Ala Ser

20                  25                  30

Thr Arg Gln Ile Asp Phe Trp Lys Pro Asp Ser Val Thr Gln Ile Lys

        35                  40                  45

Pro His Ser Thr Val Asp Phe Arg Val Lys Ala Glu Asp Ile Leu Ala

    50                  55                  60

Val Glu Asp Phe Leu Glu Gln Asn Glu Leu Gln Tyr Glu Val Leu Ile

65                  70                  75                  80

Asn Asn Leu Arg Ser Val Leu Glu Ala Gln Phe Asp Ser Arg Val Arg

                85                  90                  95

Asp Gln Val Asp Pro Arg Ser Val Ser Glu Ile Gln Leu Met His Asn

            100                 105                 110

Leu Gly Lys His Leu Asn Ser Met Glu Arg Val Glu Trp Leu Arg Lys

        115                 120                 125

Lys Leu Gln Asp Val His Asn Phe

    130                 135

<210> 19

<211> 435

<212> DNA

<213> Artificial Sequence

<220>

<223> Description of Artificial Sequence:

      PCpB-DFIAEGGGVR-hPTH fusion

<220>

<221> CDS

<222> (4)..(423)

<400> 19

cat atg cat cat agt ggt gaa cat ttc gaa ggg gag aag gtg ttc cgt    48

    Met His His Ser Gly Glu His Phe Glu Gly Glu Lys Val Phe Arg

      1               5                  10                  15

gtc aat gtt gaa gat gaa aat gac atc agc tta ctc cat gag ttg gcc    96

Val Asn Val Glu Asp Glu Asn Asp Ile Ser Leu Leu His Glu Leu Ala

                 20                  25                  30

agc acc agg cag att gac ttc tgg aaa cca gat tct gtc aca caa atc    144

Ser Thr Arg Gln Ile Asp Phe Trp Lys Pro Asp Ser Val Thr Gln Ile

             35                  40                  45

aaa cct cac agt aca gtt gac ttc cgt gtg aaa gca gaa gat att ttg    192

Lys Pro His Ser Thr Val Asp Phe Arg Val Lys Ala Glu Asp Ile Leu

         50                  55                  60

gct gtg gaa gac ttt ctg gag cag aat gaa cta caa tat gag gta ctc    240

Ala Val Glu Asp Phe Leu Glu Gln Asn Glu Leu Gln Tyr Glu Val Leu

65                  70                  75

ata aac aac ctg aga tct gtg ctc gag gct cag ttt gac agc aga gtc    288
Ile Asn Asn Leu Arg Ser Val Leu Glu Ala Gln Phe Asp Ser Arg Val
80                  85                  90                  95

cgt gac ttc atc gca gaa ggc ggt ggc gtc cgt tct gtt tct gaa atc    336
Arg Asp Phe Ile Ala Glu Gly Gly Gly Val Arg Ser Val Ser Glu Ile
                100                 105                 110

cag ctg atg cat aac ctg ggc aaa cat ctg aac tct atg gag cgt gta    384
Gln Leu Met His Asn Leu Gly Lys His Leu Asn Ser Met Glu Arg Val
            115                 120                 125

gaa tgg ctg cgt aag aag ctg cag gat gtt cac aac ttt tgatagggat cc 435
Glu Trp Leu Arg Lys Lys Leu Gln Asp Val His Asn Phe
        130                 135                 140

<210> 20
<211> 140
<212> PRT
<213> Artificial Sequence

<400> 20
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30
Thr Arg Gln Ile Asp Phe Trp Lys Pro Asp Ser Val Thr Gln Ile Lys
        35                  40                  45
Pro His Ser Thr Val Asp Phe Arg Val Lys Ala Glu Asp Ile Leu Ala
    50                  55                  60
Val Glu Asp Phe Leu Glu Gln Asn Glu Leu Gln Tyr Glu Val Leu Ile
65                  70                  75                  80
Asn Asn Leu Arg Ser Val Leu Glu Ala Gln Phe Asp Ser Arg Val Arg
                85                  90                  95
Asp Phe Ile Ala Glu Gly Gly Gly Val Arg Ser Val Ser Glu Ile Gln
            100                 105                 110
Leu Met His Asn Leu Gly Lys His Leu Asn Ser Met Glu Arg Val Glu
        115                 120                 125
Trp Leu Arg Lys Lys Leu Gln Asp Val His Asn Phe
    130                 135                 140
<210> 21
<211> 414
<212> DNA

<213> Artificial Sequence

<220>

<223> Description of Artificial Sequence: PCpB-APR-hPTH
      fusion

<220>

<221> CDS

<222> (4)..(402)

<400> 21

cat atg cat cat agt ggt gaa cat ttc gaa ggg gag aag gtg ttc cgt    48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                 20                  25                  30

agc acc agg cag att gac ttc tgg aaa cca gat tct gtc aca caa atc    144
Ser Thr Arg Gln Ile Asp Phe Trp Lys Pro Asp Ser Val Thr Gln Ile
             35                  40                  45

aaa cct cac agt aca gtt gac ttc cgt gtg aaa gca gaa gat att ttg    192
Lys Pro His Ser Thr Val Asp Phe Arg Val Lys Ala Glu Asp Ile Leu
         50                  55                  60

gct gtg gaa gac ttt ctg gag cag aat gaa cta caa tat gag gta ctc    240

Ala Val Glu Asp Phe Leu Glu Gln Asn Glu Leu Gln Tyr Glu Val Leu
65                  70                  75

ata aac aac ctg aga tct gtg ctc gag gct cag ttt gac agc aga gtc    288
Ile Asn Asn Leu Arg Ser Val Leu Glu Ala Gln Phe Asp Ser Arg Val
80                  85                  90                  95

cgt gca ccg cgg tct gtt tct gaa atc cag ctg atg cat aac ctg ggc    336
Arg Ala Pro Arg Ser Val Ser Glu Ile Gln Leu Met His Asn Leu Gly
                100                 105                 110

aaa cat ctg aac tct atg gag cgt gta gaa tgg ctg cgt aag aag ctg    384
Lys His Leu Asn Ser Met Glu Arg Val Glu Trp Leu Arg Lys Lys Leu
            115                 120                 125

cag gat gtt cac aac ttt tgatagggat cc                              414
Gln Asp Val His Asn Phe
        130

<210> 22
<211> 133
<212> PRT
<213> Artificial Sequence

<400> 22
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30
Thr Arg Gln Ile Asp Phe Trp Lys Pro Asp Ser Val Thr Gln Ile Lys
        35                  40                  45
Pro His Ser Thr Val Asp Phe Arg Val Lys Ala Glu Asp Ile Leu Ala
    50                  55                  60
Val Glu Asp Phe Leu Glu Gln Asn Glu Leu Gln Tyr Glu Val Leu Ile
65                  70                  75                  80
Asn Asn Leu Arg Ser Val Leu Glu Ala Gln Phe Asp Ser Arg Val Arg
                85                  90                  95
Ala Pro Arg Ser Val Ser Glu Ile Gln Leu Met His Asn Leu Gly Lys
            100                 105                 110
His Leu Asn Ser Met Glu Arg Val Glu Trp Leu Arg Lys Lys Leu Gln
        115                 120                 125
Asp Val His Asn Phe
    130
<210> 23
<211> 387

<212> DNA

<213> Artificial Sequence

<220>

<223> Description of Artificial Sequence: PCpB-AM-GLIP
      (R26) fusion

<220>

<221> CDS

<222> (4)..(378)

<400> 23

cat atg cat cat agt ggt gaa cat ttc gaa ggg gag aag gtg ttc cgt   48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc   96
Val Asn Val Glu Asp Glu Asn Asp Ile Ser Leu Leu His Glu Leu Ala
                 20                  25                  30

agc acc agg cag att gac ttc tgg aaa cca gat tct gtc aca caa atc   144
Ser Thr Arg Gln Ile Asp Phe Trp Lys Pro Asp Ser Val Thr Gln Ile
             35                  40                  45

aaa cct cac agt aca gtt gac ttc cgt gtg aaa gca gaa gat att ttg   192
Lys Pro His Ser Thr Val Asp Phe Arg Val Lys Ala Glu Asp Ile Leu
         50                  55                  60

gct gtg gaa gac ttt ctg gag cag aat gaa cta caa tat gag gta ctc   240
Ala Val Glu Asp Phe Leu Glu Gln Asn Glu Leu Gln Tyr Glu Val Leu
     65                  70                  75

ata aac aac ctg aga tct gtg ctc gaa gct cag ttt gac agc gct atg   288
Ile Asn Asn Leu Arg Ser Val Leu Glu Ala Gln Phe Asp Ser Ala Met
 80                  85                  90                  95

gct gaa ggg acc ttt acc agt gat gta agt tct tat ctc gag ggc caa   336
Ala Glu Gly Thr Phe Thr Ser Asp Val Ser Ser Tyr Leu Glu Gly Gln
                100                 105                 110

gct gcc cgt gaa ttc att gct tgg ctg gtg aaa ggc cga gga taaggatcc 387
Ala Ala Arg Glu Phe Ile Ala Trp Leu Val Lys Gly Arg Gly
            115                 120                 125

<210> 24
<211> 125
<212> PRT
<213> Artificial Sequence

<400> 24
Met His His Ser Gly Glu His Phe Glu Gly Glu Lys Val Phe Arg Val
  1               5                  10                  15

Asn Val Glu Asp Glu Asn Asp Ile Ser Leu Leu His Glu Leu Ala Ser
             20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Ala Met Ala
                85                  90                  95

Glu Gly Thr Phe Thr Ser Asp Val Ser Ser Tyr Leu Glu Gly Gln Ala
            100                 105                 110

Ala Arg Glu Phe Ile Ala Trp Leu Val Lys Gly Arg Gly
        115                 120                 125

<210> 25

<211> 390

<212> DNA

<213> Artificial Sequence

<220>

<223> Description of Artificial Sequence:

PCpB-RVR-GLIP(R26) fusion

<220>

<221> CDS

<222> (4)..(381)

<400> 25

cat atg cat cat agt ggt gaa cat ttc gaa ggg gag aag gtg ttc cgt    48

    Met His His Ser Gly Glu His Phe Glu Gly Glu Lys Val Phe Arg

    1               5                   10                  15

gtc aat gtt gaa gat gaa aat gac atc agc tta ctc cat gag ttg gcc    96

Val Asn Val Glu Asp Glu Asn Asp Ile Ser Leu Leu His Glu Leu Ala

                20                  25                  30

agc acc agg cag att gac ttc tgg aaa cca gat tct gtc aca caa atc    144

Ser Thr Arg Gln Ile Asp Phe Trp Lys Pro Asp Ser Val Thr Gln Ile

            35                  40                  45

aaa cct cac agt aca gtt gac ttc cgt gtg aaa gca gaa gat att ttg    192

Lys Pro His Ser Thr Val Asp Phe Arg Val Lys Ala Glu Asp Ile Leu

        50                  55                  60

gct gtg gaa gac ttt ctg gag cag aat gaa cta caa tat gag gta ctc    240

Ala Val Glu Asp Phe Leu Glu Gln Asn Glu Leu Gln Tyr Glu Val Leu

    65                  70                  75

ata aac aac ctg aga tct gtg ctc gaa gct cag ttt gac agc aga gtc    288

Ile Asn Asn Leu Arg Ser Val Leu Glu Ala Gln Phe Asp Ser Arg Val

80                    85                    90                    95

cgt gct gaa ggg acc ttt acc agt gat gta agt tct tat ctc gag ggc    336
Arg Ala Glu Gly Thr Phe Thr Ser Asp Val Ser Ser Tyr Leu Glu Gly
                100                 105                 110

caa gct gcc cgt gaa ttc att gct tgg ctg gtg aaa ggc cga gga        381
Gln Ala Ala Arg Glu Phe Ile Ala Trp Leu Val Lys Gly Arg Gly
            115                 120                 125

taaggatcc                                                          390

<210> 26
<211> 126
<212> PRT
<213> Artificial Sequence

<400> 26
Met His His Ser Gly Glu His Phe Glu Gly Glu Lys Val Phe Arg Val
 1               5                  10                  15

Asn Val Glu Asp Glu Asn Asp Ile Ser Leu Leu His Glu Leu Ala Ser
            20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Arg Val Arg
                85                  90                  95

Ala Glu Gly Thr Phe Thr Ser Asp Val Ser Ser Tyr Leu Glu Gly Gln
            100                 105                 110

Ala Ala Arg Glu Phe Ile Ala Trp Leu Val Lys Gly Arg Gly
        115                 120                 125

<210> 27
<211> 387
<212> DNA
<213> Artificial Sequence

<220>
<223> Description of Artificial Sequence:
      PCpB-RR-GLIP(R26) fusion

<220>
<221> CDS
<222> (4)..(378)

<400> 27

cat atg cat cat agt ggt gaa cat ttc gaa ggg gag aag gtg ttc cgt    48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                    20                  25                  30

agc acc agg cag att gac ttc tgg aaa cca gat tct gtc aca caa atc    144
Ser Thr Arg Gln Ile Asp Phe Trp Lys Pro Asp Ser Val Thr Gln Ile
                35                  40                  45

aaa cct cac agt aca gtt gac ttc cgt gtg aaa gca gaa gat att ttg    192
Lys Pro His Ser Thr Val Asp Phe Arg Val Lys Ala Glu Asp Ile Leu
            50                  55                  60

gct gtg gaa gac ttt ctg gag cag aat gaa cta caa tat gag gta ctc    240
Ala Val Glu Asp Phe Leu Glu Gln Asn Glu Leu Gln Tyr Glu Val Leu
        65                  70                  75

ata aac aac ctg aga tct gtg ctc gaa gct cag ttt gac agc cgt cgt    288
Ile Asn Asn Leu Arg Ser Val Leu Glu Ala Gln Phe Asp Ser Arg Arg
 80                  85                  90                  95

gct gaa ggg acc ttt acc agt gat gta agt tct tat ctc gag ggc caa    336
Ala Glu Gly Thr Phe Thr Ser Asp Val Ser Ser Tyr Leu Glu Gly Gln

                100                 105                 110

gct gcc cgt gaa ttc att gct tgg ctg gtg aaa ggc cga gga taaggatcc 387
Ala Ala Arg Glu Phe Ile Ala Trp Leu Val Lys Gly Arg Gly
            115                 120                 125

<210> 28
<211> 125
<212> PRT
<213> Artificial Sequence

<400> 28
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Arg Arg Ala
                85                  90                  95

Glu Gly Thr Phe Thr Ser Asp Val Ser Ser Tyr Leu Glu Gly Gln Ala
            100                 105                 110

Ala Arg Glu Phe Ile Ala Trp Leu Val Lys Gly Arg Gly
        115                 120                 125

<210> 29
<211> 393
<212> DNA
<213> Artificial Sequence

<220>
<223> Description of Artificial Sequence:
PCpB-EGGR-GLIP(R26) fusion

<220>
<221> CDS
<222> (4)..(384)

<400> 29
cat atg cat cat agt ggt gaa cat ttc gaa ggg gag aag gtg ttc cgt 48
Met His His Ser Gly Glu His Phe Glu Gly Glu Lys Val Phe Arg
1               5                   10                  15

gtc aat gtt gaa gat gaa aat gac atc agc tta ctc cat gag ttg gcc 96
Val Asn Val Glu Asp Glu Asn Asp Ile Ser Leu Leu His Glu Leu Ala
                20                  25                  30

agc acc agg cag att gac ttc tgg aaa cca gat tct gtc aca caa atc 144
Ser Thr Arg Gln Ile Asp Phe Trp Lys Pro Asp Ser Val Thr Gln Ile
            35                  40                  45

aaa cct cac agt aca gtt gac ttc cgt gtg aaa gca gaa gat att ttg 192
Lys Pro His Ser Thr Val Asp Phe Arg Val Lys Ala Glu Asp Ile Leu
        50                  55                  60

gct gtg gaa gac ttt ctg gag cag aat gaa cta caa tat gag gta ctc 240
Ala Val Glu Asp Phe Leu Glu Gln Asn Glu Leu Gln Tyr Glu Val Leu
    65                  70                  75

ata aac aac ctg aga tct gtg ctc gaa gct cag ttt gac agc gaa ggt 288
Ile Asn Asn Leu Arg Ser Val Leu Glu Ala Gln Phe Asp Ser Glu Gly
80                  85                  90                  95

ggt cgt gct gaa ggg acc ttt acc agt gat gta agt tct tat ctc gag 336
Gly Arg Ala Glu Gly Thr Phe Thr Ser Asp Val Ser Ser Tyr Leu Glu
                100                 105                 110

ggc caa gct gcc cgt gaa ttc att gct tgg ctg gtg aaa ggc cga gga 384
Gly Gln Ala Ala Arg Glu Phe Ile Ala Trp Leu Val Lys Gly Arg Gly
            115                 120                 125

taaggatcc 393

<210> 30

<211> 127

<212> PRT

<213> Artificial Sequence

<400> 30

Met His His Ser Gly Glu His Phe Glu Gly Glu Lys Val Phe Arg Val

1               5                   10                  15

Asn Val Glu Asp Glu Asn Asp Ile Ser Leu Leu His Glu Leu Ala Ser

            20                  25                  30

Thr Arg Gln Ile Asp Phe Trp Lys Pro Asp Ser Val Thr Gln Ile Lys

        35                  40                  45

Pro His Ser Thr Val Asp Phe Arg Val Lys Ala Glu Asp Ile Leu Ala

    50                  55                  60

Val Glu Asp Phe Leu Glu Gln Asn Glu Leu Gln Tyr Glu Val Leu Ile

65                  70                  75                  80

Asn Asn Leu Arg Ser Val Leu Glu Ala Gln Phe Asp Ser Glu Gly Gly

                85                  90                  95

Arg Ala Glu Gly Thr Phe Thr Ser Asp Val Ser Ser Tyr Leu Glu Gly

100                 105                 110

Gln Ala Ala Arg Glu Phe Ile Ala Trp Leu Val Lys Gly Arg Gly
        115                 120                 125

<210> 31
<211> 390
<212> DNA
<213> Artificial Sequence

<220>
<223> Description of Artificial Sequence:
      PCpB-QAR-GLIP(R26) fusion

<220>
<221> CDS
<222> (4)..(381)

<400> 31
cat atg cat cat agt ggt gaa cat ttc gaa ggg gag aag gtg ttc cgt    48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                 20                  25                  30

agc acc agg cag att gac ttc tgg aaa cca gat tct gtc aca caa atc 144
Ser Thr Arg Gln Ile Asp Phe Trp Lys Pro Asp Ser Val Thr Gln Ile
            35                  40                  45

aaa cct cac agt aca gtt gac ttc cgt gtg aaa gca gaa gat att ttg 192
Lys Pro His Ser Thr Val Asp Phe Arg Val Lys Ala Glu Asp Ile Leu
        50                  55                  60

gct gtg gaa gac ttt ctg gag cag aat gaa cta caa tat gag gta ctc 240
Ala Val Glu Asp Phe Leu Glu Gln Asn Glu Leu Gln Tyr Glu Val Leu
    65                  70                  75

ata aac aac ctg aga tct gtg ctc gaa gct cag ttt gac agc cag gct 288
Ile Asn Asn Leu Arg Ser Val Leu Glu Ala Gln Phe Asp Ser Gln Ala
80                  85                  90                  95

cgt gct gaa ggg acc ttt acc agt gat gta agt tct tat ctc gag ggc 336
Arg Ala Glu Gly Thr Phe Thr Ser Asp Val Ser Ser Tyr Leu Glu Gly
                100                 105                 110

caa gct gcc cgt gaa ttc att gct tgg ctg gtg aaa ggc cga gga 381
Gln Ala Ala Arg Glu Phe Ile Ala Trp Leu Val Lys Gly Arg Gly
            115                 120                 125

taaggatcc 390

<210> 32

<211> 126

<212> PRT

<213> Artificial Sequence

<400> 32

Met His His Ser Gly Glu His Phe Glu Gly Glu Lys Val Phe Arg Val

1               5                   10                  15

Asn Val Glu Asp Glu Asn Asp Ile Ser Leu Leu His Glu Leu Ala Ser

            20                  25                  30

Thr Arg Gln Ile Asp Phe Trp Lys Pro Asp Ser Val Thr Gln Ile Lys

        35                  40                  45

Pro His Ser Thr Val Asp Phe Arg Val Lys Ala Glu Asp Ile Leu Ala

    50                  55                  60

Val Glu Asp Phe Leu Glu Gln Asn Glu Leu Gln Tyr Glu Val Leu Ile

65                  70                  75                  80

Asn Asn Leu Arg Ser Val Leu Glu Ala Gln Phe Asp Ser Gln Ala Arg

                85                  90                  95

Ala Glu Gly Thr Phe Thr Ser Asp Val Ser Ser Tyr Leu Glu Gly Gln

            100                 105                 110

Ala Ala Arg Glu Phe Ile Ala Trp Leu Val Lys Gly Arg Gly

        115                 120                 125

<210> 33

<211> 395

<212> DNA

<213> Artificial Sequence

<220>

<223> Description of Artificial Sequence: PCpB-QAR-V8

      GLIP fusion

<220>

<221> CDS

<222> (4)..(384)

<400> 33

cat atg cat cat agt ggt gaa cat ttc gaa ggg gag aag gtg ttc cgt    48

    Met His His Ser Gly Glu His Phe Glu Gly Glu Lys Val Phe Arg

      1               5                  10                  15

gtc aat gtt gaa gat gaa aat gac atc agc tta ctc cat gag ttg gcc    96

Val Asn Val Glu Asp Glu Asn Asp Ile Ser Leu Leu His Glu Leu Ala

                 20                  25                  30

agc acc agg cag att gac ttc tgg aaa cca gat tct gtc aca caa atc    144

Ser Thr Arg Gln Ile Asp Phe Trp Lys Pro Asp Ser Val Thr Gln Ile

             35                  40                  45

aaa cct cac agt aca gtt gac ttc cgt gtg aaa gca gaa gat att ttg    192
Lys Pro His Ser Thr Val Asp Phe Arg Val Lys Ala Glu Asp Ile Leu
        50                  55                  60

gct gtg gaa gac ttt ctg gag cag aat gaa cta caa tat gag gta ctc    240
Ala Val Glu Asp Phe Leu Glu Gln Asn Glu Leu Gln Tyr Glu Val Leu
    65                  70                  75

ata aac aac ctg aga tct gtg ctc gag gct cag ttt gac agc cag gct    288
Ile Asn Asn Leu Arg Ser Val Leu Glu Ala Gln Phe Asp Ser Gln Ala
80                  85                  90                  95

cgt cac gtt gaa ggc acc ttc acc tct gac gtt tct tct tat ctg gaa    336
Arg His Val Glu Gly Thr Phe Thr Ser Asp Val Ser Ser Tyr Leu Glu
                100                 105                 110

ggc cag gca gca aaa gaa ttc atc gca tgg ctg gtt aaa ggc cgt ggc    384
Gly Gln Ala Ala Lys Glu Phe Ile Ala Trp Leu Val Lys Gly Arg Gly
            115                 120                 125

taataggatc c                                                       395

<210> 34
<211> 127
<212> PRT
<213> Artificial Sequence

<400> 34

Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30

Thr Arg Gln Ile Asp Phe Trp Lys Pro Asp Ser Val Thr Gln Ile Lys
        35                  40                  45

Pro His Ser Thr Val Asp Phe Arg Val Lys Ala Glu Asp Ile Leu Ala
    50                  55                  60

Val Glu Asp Phe Leu Glu Gln Asn Glu Leu Gln Tyr Glu Val Leu Ile
65                  70                  75                  80

Asn Asn Leu Arg Ser Val Leu Glu Ala Gln Phe Asp Ser Gln Ala Arg
                85                  90                  95

His Val Glu Gly Thr Phe Thr Ser Asp Val Ser Ser Tyr Leu Glu Gly
            100                 105                 110

Gln Ala Ala Lys Glu Phe Ile Ala Trp Leu Val Lys Gly Arg Gly
        115                 120                 125

<210> 35

<211> 398

<212> DNA

<213> Artificial Sequence

<220>

<223> Description of Artificial Sequence: PCpB-LVPR-V8
      GLIP fusion

<220>

<221> CDS

<222> (4)..(387)

<400> 35

cat atg cat cat agt ggt gaa cat ttc gaa ggg gag aag gtg ttc cgt    48
    Met His His Ser Gly Glu His Phe Glu Gly Glu Lys Val Phe Arg
      1               5                  10                  15

gtc aat gtt gaa gat gaa aat gac atc agc tta ctc cat gag ttg gcc    96
Val Asn Val Glu Asp Glu Asn Asp Ile Ser Leu Leu His Glu Leu Ala
                 20                  25                  30

agc acc agg cag att gac ttc tgg aaa cca gat tct gtc aca caa atc    144
Ser Thr Arg Gln Ile Asp Phe Trp Lys Pro Asp Ser Val Thr Gln Ile
             35                  40                  45

aaa cct cac agt aca gtt gac ttc cgt gtg aaa gca gaa gat att ttg    192
Lys Pro His Ser Thr Val Asp Phe Arg Val Lys Ala Glu Asp Ile Leu
         50                  55                  60

gct gtg gaa gac ttt ctg gag cag aat gaa cta caa tat gag gta ctc    240
Ala Val Glu Asp Phe Leu Glu Gln Asn Glu Leu Gln Tyr Glu Val Leu
    65                  70                  75

ata aac aac ctg aga tct gtg ctc gag gct cag ttt gac agc ctg gta    288
Ile Asn Asn Leu Arg Ser Val Leu Glu Ala Gln Phe Asp Ser Leu Val
80                  85                  90                  95

ccg cgt cac gtt gaa ggc acc ttc acc tct gac gtt tct tct tat ctg    336
Pro Arg His Val Glu Gly Thr Phe Thr Ser Asp Val Ser Ser Tyr Leu
                100                 105                 110

gaa ggc cag gca gca aaa gaa ttc atc gca tgg ctg gtt aaa ggc cgt    384
Glu Gly Gln Ala Ala Lys Glu Phe Ile Ala Trp Leu Val Lys Gly Arg
            115                 120                 125

ggc taataggatc c                                                   398
Gly

<210> 36
<211> 128
<212> PRT
<213> Artificial Sequence

<400> 36
Met His His Ser Gly Glu His Phe Glu Gly Glu Lys Val Phe Arg Val
1               5                   10                  15

Asn Val Glu Asp Glu Asn Asp Ile Ser Leu Leu His Glu Leu Ala Ser
            20                  25                  30
Thr Arg Gln Ile Asp Phe Trp Lys Pro Asp Ser Val Thr Gln Ile Lys
        35                  40                  45
Pro His Ser Thr Val Asp Phe Arg Val Lys Ala Glu Asp Ile Leu Ala
    50                  55                  60
Val Glu Asp Phe Leu Glu Gln Asn Glu Leu Gln Tyr Glu Val Leu Ile
65                  70                  75                  80
Asn Asn Leu Arg Ser Val Leu Glu Ala Gln Phe Asp Ser Leu Val Pro
                85                  90                  95
Arg His Val Glu Gly Thr Phe Thr Ser Asp Val Ser Ser Tyr Leu Glu
            100                 105                 110
Gly Gln Ala Ala Lys Glu Phe Ile Ala Trp Leu Val Lys Gly Arg Gly
        115                 120                 125

<210> 37
<211> 89
<212> PRT
<213> Homo sapiens

<400> 37

Glu Leu Thr Gly Gln Arg Leu Arg Glu Gly Asp Gly Pro Asp Gly Pro

1               5                   10                  15

Ala Asp Asp Gly Ala Gly Ala Gln Ala Asp Leu Glu His Ser Leu Leu

            20                  25                  30

Val Ala Ala Glu Lys Lys Asp Glu Gly Pro Tyr Arg Met Glu His Phe

        35                  40                  45

Arg Trp Gly Ser Pro Pro Lys Asp Lys Arg Tyr Gly Gly Phe Met Thr

    50                  55                  60

Ser Glu Lys Ser Gln Thr Pro Leu Val Thr Leu Phe Lys Asn Ala Ile

65                  70                  75                  80

Ile Lys Asn Ala Tyr Lys Lys Gly Glu

                85

<210> 38

<211> 296

<212> DNA

<213> Artificial Sequence

<220>

<223> Description of Artificial Sequence: Synthetic BLT

      Gene

<220>

<221> CDS

<222> (19)..(285)

<400> 38

gtatctctcg agaaaaga gaa ctg acc ggt cag cgt ctg cgc gaa ggt gac 51

Glu Leu Thr Gly Gln Arg Leu Arg Glu Gly Asp

1               5                   10

ggt ccg gac ggt ccg gct gac gac ggt gct ggt gct cag gca gat ctg 99

Gly Pro Asp Gly Pro Ala Asp Asp Gly Ala Gly Ala Gln Ala Asp Leu

            15                  20                  25

gaa cac tcc ctg ctg gtt gct gca gag aag aaa gac gaa ggt ccg tac 147

Glu His Ser Leu Leu Val Ala Ala Glu Lys Lys Asp Glu Gly Pro Tyr

        30                  35                  40

cgt atg gaa cac ttc cgt tgg ggt tcc ccg ccg aaa gac aag cgt tac 195

Arg Met Glu His Phe Arg Trp Gly Ser Pro Pro Lys Asp Lys Arg Tyr

    45                  50                  55

ggt ggt ttc atg acc tcc gag aaa tcc cag acc ccg ctg gtt act ctg 243

Gly Gly Phe Met Thr Ser Glu Lys Ser Gln Thr Pro Leu Val Thr Leu

60                  65                  70                  75

ttc aag aac gct atc atc aag aac gcg tac aag aaa ggt gaa 285

Phe Lys Asn Ala Ile Ile Lys Asn Ala Tyr Lys Lys Gly Glu

                80                  85

taataggatc c 296

<210> 39

<211> 89

<212> PRT

<213> Artificial Sequence

<400> 39

Glu Leu Thr Gly Gln Arg Leu Arg Glu Gly Asp Gly Pro Asp Gly Pro

1               5                   10                  15

Ala Asp Asp Gly Ala Gly Ala Gln Ala Asp Leu Glu His Ser Leu Leu

            20                  25                  30

Val Ala Ala Glu Lys Lys Asp Glu Gly Pro Tyr Arg Met Glu His Phe

        35                  40                  45

Arg Trp Gly Ser Pro Pro Lys Asp Lys Arg Tyr Gly Gly Phe Met Thr

    50                  55                  60

Ser Glu Lys Ser Gln Thr Pro Leu Val Thr Leu Phe Lys Asn Ala Ile

65                  70                  75                  80

Ile Lys Asn Ala Tyr Lys Lys Gly Glu

                85

<210> 40

<211> 92

<212> DNA

<213> Artificial Sequence

<220>

<223> Description of Artificial Sequence: Lars 792

      Oligonucleotide

<400> 40

tggaacactc cctgctggtt gctgcagaga agaaagacga aggtccgtac cgtatggaac 60

acttccgttg gggttccccg ccgaaagaca ag                               92

<210> 41

<211> 92

<212> DNA

<213> Artificial Sequence

<220>

<223> Description of Artificial Sequence: Lars 793

      oligonucleotide

<400> 41

gtaacgcttg tctttcggcg gggaacccca acggaagtgt tccatacggt acggaccttc 60

gtctttcttc tctgcagcaa ccagcaggga gt                                    92

<210> 42

<211> 102

<212> DNA

<213> Artificial Sequence

<220>

<223> Description of Artificial Sequence: Lars 794

      oligonucleotide

<400> 42

cgttacggtg gtttcatgac ctccgagaaa tcccagaccc cgctggttac tctgttcaag 60

aacgctatca tcaagaacgc gtacaagaaa ggtgaataat ag                        102

<210> 43

<211> 99

<212> DNA

<213> Artificial Sequence

<220>

<223> Description of Artificial Sequence: Lars 795

      oligonucleotide

<400> 43
gatcctatta ttcacctttc ttgtacgcgt tcttgatgat agcgttcttg aacagagtaa 60
ccagcggggt ctgggatttc tcggaggtca tgaaaccac 99

<210> 44
<211> 90
<212> DNA
<213> Artificial Sequence

<220>
<223> Description of Artificial Sequence: Lars 796
      oligonucleotide

<400> 44
tcgagaaaag agaactgacc ggtcagcgtc tgcgcgaagg tgacggtccg gacggtccgg 60
ctgacgacgg tgctggtgct caggcagatc 90

<210> 45
<211> 92
<212> DNA
<213> Artificial Sequence

<220>
<223> Description of Artificial Sequence: Lars 797

oligonucleotide

<400> 45
gttccagatc tgcctgagca ccagcaccgt cgtcagccgg accgtccgga ccgtcacctt 60

cgcgcagacg ctgaccggtc agttctcttt tc 92

<210> 46
<211> 66
<212> DNA
<213> Artificial Sequence

<220>
<223> Description of Artificial Sequence: MM402
oligonucleotide

<400> 46
gcggccgcct cgaggctcag tttgacagcc tggtaccgcg tgaactgacc ggtcagcgtc 60

tgcgcg 66

<210> 47
<211> 45
<212> DNA
<213> Artificial Sequence

<220>

<223> Description of Artificial Sequence: MM403

      oligonucleotide

<400> 47

gcggccgcgg atccctatta ttcacctttc ttgtacgcgt tcttg                45

<210> 48

<211> 78

<212> DNA

<213> Artificial Sequence

<220>

<223> Description of Artificial Sequence: MM105

      oligonucleotide

<400> 48

tcgaggctca gtttgacagc agagtccgtt ctgtttctga aatccagctg atgcataacc 60

tgggcaaaca tctgaact                                              78

<210> 49

<211> 67

<212> DNA

<213> Artificial Sequence

<220>

<223> Description of Artificial Sequence: MM106

      oligonucleotide

<400> 49

atgtttgccc aggttatgca tcagctggat ttcagaaaca gaacggactc tgctgtcaaa 60

ctgagcc                                                           67

<210> 50

<211> 60

<212> DNA

<213> Artificial Sequence

<220>

<223> Description of Artificial Sequence: MM107

      oligonucleotide

<400> 50

ctatggagcg tgtagaatgg ctgcgtaaga agctgcagga tgttcacaac ttttgatagg 60

<210> 51

<211> 71

<212> DNA

<213> Artificial Sequence

<220>

<223> Description of Artificial Sequence: MM108
      oligonucleotide

<400> 51
gatccctatc aaaagttgtg aacatcctgc agcttcttac gcagccattc tacacgctcc 60

atagagttca g                                                      71

<210> 52
<211> 58
<212> DNA
<213> Artificial Sequence

<220>
<223> Description of Artificial Sequence: MM139
      oligonucleotide

<400> 52
tcgaggctca gtttgactcg atcgaaggcc gttctgtttc tgaaatccag ctgatgca   58

<210> 53
<211> 50
<212> DNA
<213> Artificial Sequence

<220>

<223> Description of Artificial Sequence: MM140

<400> 53

tcagctggat ttcagaaaca gaacggcctt cgatcgagtc aaactgagcc 50

## Description

This application claims the benefit of U.S. Provisional Application No. 60/113,058, filed December 21, 1998.

This invention relates to recombinant DNA processes. In particular the invention pertains to recombinant methods for making beta-lipotropin and other peptides.

Proopiomelanocortin (POMC) is a neuropeptide precursor molecule which is translocated to secretory pathways within neuroendocrine cells. POMC is cleaved by the action of specific endopeptidases to yield peptides such as adrenocorticotrophic hormone (ACTH), Beta-lipotropin (BLT), Beta-endorphin, and Melanocyte Stimulating Hormone (MSH). The processing of POMC into one or more specific peptides occurs in a tissue and cell specific manner (See generally, M. Castro and E. Morrison, Crit. Rev. Neurobiol., 11, 35-57, 1997; Roberts, J. L. and Herbert, E., Proc Nat Acad Sci, 74, 4826 (1977); Roberts, J. L. and Herbert, E., Proc.Nat.Acad.Sci 74, 5300 (1977); Mains, et al., Proc.Nat.Acad.Sci 74, 3014 (1977)). POMC is produced mainly in the pituitary gland and hypothalamus. Post-translational processing of POMC in the anterior pituitary produces ACTH and BLT. On the other hand, the major products of the intermediate pituitary are (-MSH, CLIP, (-lipotropin, (-endorphin, and (-MSH, while in the hypothalamus, POMC is processed primarily into (-MSH and (-endorphin.

POMC-derived peptides perform a variety of important roles in metabolic and physiological regulation. For example, ACTH, a 39 amino acid peptide, stimulates secretion of glucocorticoids from the adrenal cortex. The MSH's, on the other hand, stimulate melanin synthesis by melanocytes in the skin, and also appear to be involved in fat metabolism. (-endorphin derives from the carboxyl end of BLT (viz. Residues 59 to 89 of the human sequence), and possesses analgesic activity that is antagonized by naloxone, a known antagonist for morphine. Thus, POMC-derived peptide hormones have diverse roles in physiologic and metabolic regulation.

Beta-lipotropin and other small peptides are difficult to purify from whole tissues. While solid-phase synthetic methods are useful in producing such peptides, these methods are limited by relatively small production capacities. Recombinant methods offer the best option for producing large quantities of such peptides. However, designing an optimal recombinant system presents many challenges including high level expression and a convenient purification method. In many instances, fusion protein systems provide the best option. However, fusion systems can be problematic, for example, in only achieving low level expression, sensitivity to proteolysis, and protein insolubility.

Disclosed herein are fusion protein methods for producing BLT and other peptides in *E.coli* and yeast based on using the propeptide sequence of procarboxypeptidase B (PCpB). Unlike other fusion systems, the procarboxypeptidase system has distinct advantages, including high level expression of the fusion protein, and solubility and heat stability of the fusion protein. In one embodiment of the present invention, a fusion protein comprising the propeptide sequence of procarboxypeptidase B and a peptide of interest is produced in a host cell. In *E.coli,* the PCpB fusion protein can be recovered from cell lysates under conditions that inhibit proteolysis using conventional purification methods. In *Pichia pastoris,* the procarboxypeptidase fusion partner is cleaved upon secretion of the fusion protein from the cell, thereby releasing native peptide into the culture medium.

The present invention provides fusion proteins comprising the propeptide of PCpB fused to a heterologous protein, for example, beta-lipotropin (BLT), and other proteins, nucleic acids encoding same, and recombinant methods for producing BLT and/or other peptides.

Disclosed herein are fusion protein methods for producing proteins in *E.coli* and yeast or other suitable host cells. While the method of the invention is generally applicable to the synthesis of any protein, the method is described specifically using BLT as an non-exclusive example of the invention. The examples and description vis-à-vis BLT are exemplary only, and are not intended to limit the scope of the invention.

A BLT fusion protein contains a recognition site for a specific protease, or site that imparts chemical sensitivity. Said recognition site provides a means to separate the propeptide fusion partner from BLT. In *Pichia pastoris,* the fusion protein is cleaved upon secretion of the fusion protein from the cell such that native BLT can be recovered intact from the culture medium. The PCpB propeptide sequence (SEQ ID NO:1) provides solubility to fusion proteins.

In one embodiment, the present invention relates to a fusion protein comprising a propeptide sequence of procarboxypeptidase B and an heterologous protein fused thereto.

In another embodiment the present invention relates to a fusion protein having an amino acid sequence which is selected from the group consisting of SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36.

In still another embodiment the present invention relates tc an isolated nucleic acid compound comprising a nucleic acid that encodes a procarboxypeptidase B fusion partner, comprising beta lipotropin or other peptide.

In still another embodiment the present invention relates tc an isolated nucleic acid compound identified herein as SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35.

In still another embodiment the present invention relates to a vector comprising an isolated nucleic acid compound of the invention.

In yet another embodiment the present invention relates to a vector comprising an isolated nucleic acid of the invention, wherein said isolated nucleic acid compound is operably-linked to a promoter sequence.

In another embodiment the present invention relates to a host cell containing a vector of the present invention.

In yet another embodiment the present invention relates to a method for constructing a recombinant host cell having the potential to express a fusion protein comprising beta-lipotropin, or other peptide, said method comprising introducing into said host cell by any suitable means a vector of the present invention.

In still another embodiment the present invention relates to a method for expressing beta-lipotropin or other peptide in a recombinant host cell, said method comprising culturing said recombinant host cell under conditions suitable for gene expression.

In another embodiment the present invention relates to a process for preparing beta-lipotropin or other peptide comprising:
a. transforming a suitable host with an expression vector wherein said vector encodes a PCpB fusion protein comprising the peptide of interest;
b. culturing said transformed host under conditions that enable expression of said peptide;
c. purifying said peptide by any suitable means.

### Definitions

"AA" refers to amino acid.
"NA" refers to nucleic acid.
"hPTH" refers to human parathyroid hormone.
"GLIP" refers to glucagon-like insulinotropic peptide.
"BLT" refers to beta-lipotropin. The human BLT comprises 89 amino acid residues (SEQ ID NO:8). The BLT protein has been characterized in a variety of organisms and the amino acid sequences determined in a variety of organisms including human, mouse, ovine, and porcine (Takahashi et al. *FEBS Lett.* 135, 97-102 (1981); Li & Chung, *Nature,* 260, 622-24 (1976); and elephant (Li *et al. Int. J. Pept. Prot. Res.* 32, 573-78, 1988); as well as other mammals, all of which are hereby incorporated by reference.

The term "beta-lipoprotein fusion protein" or "BLT fusion protein" refers to a class of hybrid recombinant protein molecules comprising BLT, that may be produced in *E.coli* or other cell type and from which can be generated BLT, or a BLT fragment through specific proteolysis or chemical cleavage. Exemplary BLT fusion proteins include those specified herein as SEQ ID NO:3 through SEQ ID NO:6.

The terms "complementary" or "complementarity" as used herein refer to the capacity of purine and pyrimidine nucleotides to associate through hydrogen bonding to form double stranded nucleic acid molecules. The following base pairs are related by complementarity: guanine and cytosine; adenine and thymine; and adenine and uracil. As used herein, "complementary" means that the aforementioned relationship applies to substantially all base pairs comprising two single-stranded nucleic acid molecules over the entire length of said molecules. "Partially complementary" refers to the aforementioned relationship in which one of two single-stranded nucleic acid molecules is shorter in length than the other such that a portion of one of the molecules remains single-stranded.

"Fragment thereof" refers to a fragment, piece, or subregion of a peptide, or a nucleic acid encoding a peptide, such that the fragment comprises 2 (two) or more contiguous amino acids, or alternatively about 5 to 14 amino acids, or greater; or 10 or more nucleotides that are contiguous in the parent peptide or nucleic acid molecule. Fragment thereof may or may not retain biological activity. For example, a fragment of a peptide disclosed herein could be used as an antigen to raise a specific antibody against the parent peptide from which the fragment is derived. When referring to a nucleic acid molecule, "fragment thereof" refers to 10 or more contiguous nucleotides, derived from the parent nucleic acid, and also, owing to the genetic code, to the complementary sequence. For example if the fragment entails the sequence 5'-GGCCAGCTAG-3', then "fragment thereof" would also include the complementary sequence, 3'-CCGGTCGATC-5'.

The term "fusion protein" denotes a hybrid protein molecule not found in nature comprising a translational fusion or enzymatic fusion in which two or more different proteins or fragments thereof are covalently linked on a single polypeptide chain. For example, a fusion protein comprises the propeptide of procarboxypeptidase B fused to a protein or peptide comprising an heterologous amino acid sequence.

"Fusion partner" refers to a sequence of amino acids that is fused to a protein of interest, and constituting a portion of a fusion protein, e.g. the propeptide of procarboxypeptidase B identified herein as SEQ ID NO:2.

"Host cell" refers to any eucaryotic or procaryotic cell that is suitable for propagating and/or expressing a cloned gene contained on a vector that is introduced into said host cell by, for example, transformation or transfection, or the like.

The term "hybridization" as used herein refers to a process in which a single-stranded nucleic acid molecule joins with a complementary strand through nucleotide base pairing. "Selective hybridization" refers to hybridization under conditions of high stringency. The degree of hybridization depends upon, for example, the degree of homology, the stringency of hybridization, and the length of hybridizing strands.

"Isolated nucleic acid compound" refers to any RNA or DNA sequence, however constructed or synthesized, which is locationally distinct from its natural location, e.g. in a cell.

The term "linker peptide" refers to a short amino acid sequence, typically from 2 amino acid residues to about 10 amino acid residues inserted between a fusion partner and protein of interest in a fusion protein, said linker peptide providing a site for enzymatic or chemical cleavage to separate said fusion partner from said protein of interest.

The term "plasmid" refers to an extrachromosomal genetic element. The plasmids disclosed herein are commercially available, publicly available on an unrestricted basis, or can be constructed from readily available plasmids in accordance with published procedures.

The terms "protein" and "peptide" are used interchangeably throughout, referring to two or more amino acid residues covalently linked by peptide bonding. In some instances these terms describe amino acid biopolymers comprising greater than 10 and up to about 1000 to 2000 amino acid residues linked by peptide bonding.

"Recombinant DNA cloning vector" as used herein refers to any autonomously replicating agent, including, but not limited to, plasmids and phages, comprising a DNA molecule to which one or more additional DNA segments can or have been incorporated.

The term "recombinant DNA expression vector" or "expression vector" as used herein refers to any recombinant DNA cloning vector, for example a plasmid or phage, in which a promoter and other regulatory elements are present thereby enabling transcription of an inserted DNA, which may encode a protein.

"Substantially pure" in reference to a protein means that said protein is separated from other cellular and non-cellular components, including other protein molecules. A substantially pure preparation is at least 85% pure; and preferably about at least 95% pure. A "substantially pure" protein could be prepared by any number of suitable methods including, for example, the IMAC protein purification method (U.S. Patent No. 4,569,794) herein incorporated by reference.

All references cited in this specification are herein incorporated by reference.

Beta-lipotropin was isolated in 1964 from ovine pituitary glands, and its primary structure reported the following year (Li *et. al. Nature,* 208, 1093, 1965). Since then the primary sequence of sheep, bovine, ovine, mouse, porcine, guinea pig, rat, elephant, and human BLTs have been reported. (*See e.g.* Lohmar and Li, *Biochim. Biophys. Acta,* 147, 381, 1967; Li and Chung, *Nature,* 260, 622, 1976; Drouin and Goodman, *Nature,* 288, 619, 1980; Li *et al. Int. J. Pept. Prot. Res.* 32, 573-78, 1988; Takahashi et al. FEBS Lett. 135, 97-102 (1981); all of which are herein incorporated by reference). The sequence data reveal that the carboxy terminus of BLT is highly conserved across species. However, considerable sequence differences occur at the amino terminal end of BLT molecules from different species.

The present invention relates to fusion proteins, for example BLT fusion proteins, comprising human BLT (SEQ ID NO:37), or BLT from another species, or a functional variant thereof, or a functional fragment thereof. Exemplary BLT fusion protein amino acid and nucleic acid sequences are disclosed herein as SEQ ID NO:3 through SEQ ID NO:6.

Betalipotropin protein was produced initially by chemical synthesis. However, this method was not useful for generating large quantities of BLT protein. In this invention, we describe a method by which BLT and other proteins can be produced in bacterial or fungal hosts in the form of fusion proteins. The fusion proteins of the present invention are protected from proteolytic degradation and allow for recovery of intact BLT by methods described below.

In *E.coli,* BLT is produced as a fusion protein which can be recovered from cell lysates in the presence of high concentrations of urea or other suitable denaturant, for example, sodium dodecyl sulfate, by conventional purification methods. The fusion protein contains a recognition site for specific proteases or chemical cleavage which enable the separation of the fusion partner from BLT. In *Pichia pastoris,* secretion of a fusion protein results in cleavage and release of a heterologous protein which can be detected and recovered from the culture medium.

The invention disclosed herein applies to any fusion system that can be expressed in bacterial or fungal hosts. A suitable fusion system contains site(s) that are placed between a heterologous protein, for example BLT, and the terminal fusion partner. Such site(s) provide recognition sequences for scission by proteases or by chemical cleavage.

The present invention also relates to fungal fusion systems that include fusion partners such as the alpha mating factor, propeptide, human serum albumin, and any other sequence that promotes expression and secretion of the fusion protein, and subsequent cleavage (during secretion from the fungal cell) to release heterologous protein, e.g. BLT, into the culture medium.

**Table 1**

| SEQUENCE LISTING IDENTIFIER | | |
|---|---|---|
| SEQ ID NO. | DESCRIPTION | Kind |
| 1 | Propeptide of PCpB | NA |
| 2 | Propeptide of PCpB | AA |
| 3 | AMF-PCpB-BLT fusion | NA |
| 4 | AMF-PCpB-BLT fusion | AA |
| 5 | PCpB/LVPR/BLTfusion | NA |
| 6 | PCpB/LVPR/BLT fusion | AA |
| 7 | PCpB/RVR/hPTH (1-34) | NA |
| 8 | PCpB/RVR/hPTH (1-34) | AA |
| 9 | PCpB/ALY/hPTH (1-34) | NA |
| 10 | PCpB/ALY/hPTH (1-34) | AA |
| 11 | PCpB/IEGR/hPTH (1-34) | NA |
| 12 | PCpB/IEGR/hPTH (1-34) | AA |
| 13 | PCpB/LVPR/hPTH (1-34) | NA |
| 14 | PCpB/LVPR/hPTH (1-34) | AA |
| 15 | PCpB/VIPR/hPTH (1-34) | NA |
| 16 | PCpB/VIPR/hPTH (1-34) | AA |
| 17 | PCpB/DQVDPR/hPTH (1-34) | NA |
| 18 | PCpB/DQVDPR/hPTH (1-34) | AA |
| 19 | PCpB/DFIAEGGGVR/hPTH (1-34) | NA |
| 20 | PCpB/DFIAEGGGVR/hPTH (1-34) | AA |
| 21 | PCpB/APR/hPTH (1-34) | NA |
| 22 | PCpB/APR/hPTH (1-34) | AA |
| 23 | PCpB/AM/GLIP(R26) | NA |
| 24 | PCpB/AM/GLIP(R26) | AA |
| 25 | PCpB/RVR/GLIP(R26) | NA |
| 26 | PCpB/RVR/GLIP(R26) | AA |
| 27 | PCpB/RR/GLIP(R26) | NA |
| 28 | PCpB/RR/GLIP(R26) | AA |
| 29 | PCpB/EGGR/GLIP(R26) | NA |
| 30 | PCpB/EGGR/GLIP(R26) | AA |
| 31 | PCpB/QAR/GLIP(R26) | NA |
| 32 | PCpB/QAR/GLIP(R26) | AA |
| 33 | PCpB/QAR/V8-GLIP | NA |
| 34 | PCpB/QAR/V8-GLIP | AA |
| 35 | PCpB/LVPR/V8-GLIP | NA |
| 36 | PCpB/LVPR/V8-GLIP | AA |
| 37 | Human BLT | AA |
| 38 | Synthetic nucleic acid | NA |
| | encoding human BLT | |
| 39 | encoding human BLT | AA |
| 40 | Lars 792 | NA |
| 41 | Lars 793 | NA |
| 42 | Lars 794 | NA |
| 43 | Lars 795 | NA |
| 44 | Lars 796 | NA |
| 45 | Lars 797 | NA |
| 46 | MM 402 | NA |
| 47 | MM 403 | NA |
| 48 | MM 105 | NA |
| 49 | MM 106 | NA |
| 50 | MM 107 | NA |
| 51 | MM 108 | NA |
| 52 | MM 139 | NA |
| 53 | MM 140 | NA |

Those skilled in the art will recognize that a nucleic acid encoding BLT, or a BLT fusion, or other fusion protein or peptide can be obtained by a plurality of recombinant DNA techniques including, for example, polymerase chain reaction (PCR) amplification, or de novo DNA synthesis.(See e.g., T. Maniatis et al. Molecular Cloning: A Laboratory Manual, 2d Ed. Chap. 14 (1989)).

For example, oligonucleotide primers targeted to the 3' and 5' ends of SEQ ID NO:3 can be used for PCR amplification of alpha mating factor-PCpB-BLT. See e.g. PCR Protocols: A Guide to Method and Application, Ed. M. Innis et al., Academic Press (1990). A PCR amplification comprises template DNA, suitable enzymes, primers, and buffers, and is conveniently carried out in a DNA Thermal Cycler (Perkin Elmer Cetus, Norwalk, CT). A positive result is determined by detecting an appropriately-sized DNA fragment following agarose gel electrophoresis.

Skilled artisans will recognize that the proteins and fragments, or functional fragments thereof, of the present invention can be synthesized by a number of different methods, such as chemical methods well known in the art, including solid phase peptide synthesis or recombinant methods. Both methods are described in U.S. Patent 4,617,149, incorporated herein by reference.

The proteins of the present invention can also be produced by recombinant DNA methods. Expression of a cloned nucleic acid encoding BLT, or BLT fusion, or other fusion protein can be carried out in a variety of suitable host cells, well known to those skilled in the art. For this purpose, a nucleic acid encoding a BLT fusion protein is introduced into a host cell by any suitable means, well known to those skilled in the art. For expression in procaryotes chromosomal integration is within the scope of the present invention, though it is preferred that the sequence be cloned into a suitable extra-chromosomally maintained expression vector so that the coding region of the BLT gene is operably-linked to a constitutive or inducible promoter.

The basic steps in the recombinant production of the BLT protein are:
a) constructing a natural, synthetic or semi-synthetic DNA encoding a BLT fusion protein thereof;
b) integrating said DNA into an expression vector in a manner suitable for expressing said protein;
c) transforming or otherwise introducing said vector into an appropriate eucaryotic or prokaryotic host cell forming a recombinant host cell,
d) culturing said recombinant host cell in a manner amenable to expression of said protein; and
e) recovering and substantially purifying said protein by any suitable means.

Human beta-lipotropin (BLT) is an 89-amino acid hormone (SEQ ID NO:37) produced by the pituitary in the form of a precursor protein that undergoes post-translational processing to generate several bioactive hormones including BLT. Because BLT is small and contains proteolytically sensitive sites, protein was produced initially by chemical synthesis. However, chemical synthesis is not useful for generating large quantities of protein. This invention describes a method by which BLT can be produced in bacterial or fungal expression hosts in the form of fusion proteins that are protected from proteolytic degradation, and allow for recovery of intact protein by methods described below.

A BLT fusion protein containing an amino-terminal procarboxypeptidase sequence can be expressed at high levels in *E.coli.* A cleavage site for thrombin (e.g. LVPR), or other suitable protease or chemical cleavage site, is placed between the fusion partner and BLT.

The present invention enables BLT to be produced as a fusion protein which can be recovered from cell lysates under conditions that inhibit proteolysis, for example in the presence of denaturants such as urea, by conventional purification methods. The fusion protein contains a recognition site for specific proteases or chemical cleavage, used to separate the fusion partner from BLT. Examples of suitable proteases include Factor Xa, thrombin and enterokinase. Agents for chemical cleavage include cyanogen bromide, acid, etc. In *Pichia pastoris,* the fusion protein is cleaved upon secretion from the cell in such a way that native BLT can be detected and recovered intact from the culture medium.

The present method offers a production process for BLT and other proteins. Unlike chemical synthesis, the process described herein can be scaled up to produce large amounts of BLT and other proteins.

Procaryotes may be employed in the production of recombinant BLT protein. For example, the Escherichia coli K12 strain 294 (ATCC No. 31446) or RV308 (ATCC No. 31608) are particularly useful for expression of foreign proteins in a procaryotic cell. Other strains of *E.coli,* bacilli such as *Bacillus subtilis,* enterobacteriaceae such as *Salmonella typhimurium* or *Serratia marcescans,* various *Pseudomonas* species and other bacteria, such as *Streptomyces,* may also be employed as host cells in the cloning and expression of the recombinant proteins of this invention.

Promoter sequences suitable for driving the expression of genes in procaryotes include β-lactamase [*e.g.* vector pGX2907, ATCC 39344, contains a replicon and β-lactamase gene], lactose systems [Chang et al., *Nature*_(London), 275:615 (1978); Goeddel et al., *Nature* (London), 281:544 (1979)], alkaline phosphatase, and the tryptophan (trp) promoter system [vector pATH1 (ATCC 37695)], which is designed to facilitate expression of an open reading frame as a trpE fusion protein under the control of the trp promoter. Other suitable promoters include the T7 and pL promoters. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably-linked to the DNA encoding the desired polypeptides. These examples are illustrative rather than limiting.

In one embodiment of a suitable fusion system, a recognition site is placed between BLT and a fusion partner, wherein, for example, the fusion partner is placed at the amino terminal end of BLT. A suitable site can be a recognition sequence for a protease, or a site that is susceptible to chemical cleavage.

Examples of fusion partners that are useful in fungal systems such as *Pichia pastoris* include human serum albumin (HAS), or any other sequence that is expressed and secreted in *Pichia* such that BLT can be recovered from the culture medium. Other suitable fusion partners include the yeast alpha mating factor (AMF), the carboxypeptidase B propeptide (e.g. SEQ ID NO:2), and any other sequence that promotes expression, secretion, and subsequent cleavage (during secretion) of the fusion protein to release native BLT into the culture medium.

It is often observed in the production of certain peptides in recombinant systems that expression as a fusion protein prolongs the lifespan, increases the yield of the desired peptide, and/or provides a convenient means of purifying the protein. This is particularly relevant when expressing mammalian proteins in procaryotic hosts. A variety of peptidases (e.g. enterokinase and thrombin) which cleave a polypeptide at specific sites or digest the peptides from the amino or carboxy termini (*e.g.* diaminopeptidase) of the peptide chain are known.
Furthermore, particular chemicals (*e.g.* cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. *See e.g.,* P. Carter, "Site Specific Proteolysis of Fusion Proteins", Chapter 13, in Protein Purification: From Molecular Mechanisms to Large Scale Processes, American Chemical Society, Washington, D.C. (1990).

In addition to procaryotes, a variety of amphibian expression systems such as frog oocytes, and mammalian cell systems can be used. The choice of a particular host cell depends to some extent on the particular expression vector used. Exemplary mammalian host cells suitable for use in the present invention include HepG-2 (ATCC HB 8065), CV-1 (ATCC CCL 70), LC-MK₂ (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1484), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2-b-globin, in which the SV40 promoter drives transcription of an inserted gene. These vectors are widely available from sources such as the American Type Culture Collection (ATCC), 12301 Parklawn Drive, Rockville, Maryland, 20852, or the Northern Regional Research Laboratory (NRRL), 1815 N. University Street, Peoria, Illinois, 61604.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes.

Transfection of mammalian cells with vectors can be performed by a plurality of well known processes including, but not limited to, protoplast fusion, calcium phosphate coprecipitation, electroporation and the like. *See, e.g.,* Maniatis *et al., supra.*

Some viruses also make appropriate vectors. Examples include the adenoviruses, the adeno-associated viruses, the vaccinia virus, the herpes viruses, the baculoviruses, and the rous sarcoma virus, as described in U.S. Patent 4,775,624, incorporated herein by reference.

Eucaryotic microorganisms such as yeast and other fungi are suitable host cells. The yeasts *Saccharomyces cerevisiae* and *Pichia pastoris* are the preferred eucaryotic microorganisms. Other yeasts such as *Kluyveromyces lactis* are also suitable. For expression in *Saccharomyces,* the plasmid YRp7 (ATCC-40053), for example, may be used. *See, e.g.,* L. Stinchcomb *et al., Nature,* 282, 39 (1979); J. Kingsman *et al., Gene,* 7, 141 (1979); S. Tschemper *et al., Gene,* 10, 157 (1980). Plasmid YRp7 contains the TRP1 gene which provides a selectable marker for use in a trp1 auxotrophic mutant.

Other embodiments of the present invention comprise isolated nucleic acid sequences that encode BLT, or fragment thereof. As skilled artisans will recognize, the amino acid compounds of the invention can be encoded by a multitude of different nucleic acid sequences. Because these alternative nucleic acid sequences would encode the same amino acid sequences, the present invention further comprises these alternate nucleic acid sequences.

The BLT encoding nucleic acids of the invention may also be produced by chemical synthetic methods. The synthesis of nucleic acids is well known in the art. *See, e.g.,* E.L. Brown, R. Belagaje, M.J. Ryan, and H.G. Khorana, *Methods in Enzymology,* 68:109-151 (1979). Fragments of the DNA sequence corresponding to BLT could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire gene. Alternatively, phosphotriester chemistry may be employed to synthesize the nucleic acids of this invention. (*See, e.g.,* M.J. Gait, ed., Oligonucleotide Synthesis, A Practical Approach, (1984)).

The invention provides a convenient method to produce peptides in *E.coli.* Peptides generally cannot be produced by direct biosynthesis in *E.coli* because of rapid digestion by intracellular proteases. Moreover, many fusion proteins form insoluble granules in *E.coli.* Therefore, significant quantities of the fusion peptides can accumulate in *E.coli* cells as insoluble granules. Additionally, release of the desired peptide from a fusion protein can be difficult because such granules are not soluble under mild conditions, and most site specific proteases are denatured or not active under the harsher conditions needed to dissolve the granules.

Procarboxypeptidase fusion proteins produced by the present invention solve this problem by remaining soluble in *E.coli.* The fusion proteins of the present invention are further unique in being resistant to heat denaturation. Most *E.coli* proteins are denatured and precipitated by heat. Thus, heat precipitation provides a convenient method to purify the fusion proteins of the invention from other cellular proteins. In one embodiment of this aspect, cells are lysed, extracts heated, and the fusion protein recovered in the supernatant. In another embodiment, intact cells are heated thereby lysing a fraction of cells. The fusion protein can then be recovered in the soluble fraction.

The invention further comprises a recombinant gene that codes for a fusion protein containing the propeptide sequence of porcine procarboxypeptidase B (PCpB), a linker peptide that contains a specific cleavage site for enzymatic or chemical cleavage, and a peptide of interest. Several preferred linker sequences are used to facilitate optimal cleavage using a variety of site specific proteases. For example, suitable cleavage sites include Factor Xa, several different sequences recognized by trypsin, and several different sequences cleaved by thrombin, all of which are known to the skilled artisan.

The invention may be used as a method to prepare pharmaceutically active peptides. For example, a plasmid coding for a fusion protein is prepared by inserting a DNA fragment encoding the desired peptide into the parental plasmid, which contains the coding sequence for the carboxypeptidase propeptide. The plasmid additionally comprises a linker peptide sequence that provides a site for protease or chemical cleavage.

Another aspect of the present invention relates to recombinant DNA cloning vectors and expression vectors comprising the nucleic acids of the present invention. The preferred nucleic acid vectors are those which comprise DNA. Recombinant DNA vectors for expressing BLT and other proteins are shown in Table 2.

**Table 2.**

| Plasmids coding for Propeptide fusions | | |
|---|---|---|
| Plasmid | Protease Cleavage Site | Fusion Peptide |
| pHMM66 | Trypsin | RVR/hPTH(1-34) |
| pHMM67 | Thrombin | LVPR/hPTH(1-34) |
| pHMM68 | Chymotrypsin | ALY/hPTH(1-34) |
| pHMM69 | Factor Xa | IEGR/hPTH(1-34) |
| pHMM96 | Thrombin or Trypsin | VIPR/hPTH(1-34) |
| pHMM97 | Thrombin or Trypsin | DQVDPR/hPTH(1-34) |
| pHMM98 | Thrombin or Trypsin | DFIAEGGGVR/hPTH(1-34) |
| pHMM99 | Thrombin or Trypsin | APR/hPTH(1-34) |
| pHDM279 | | AM/GLIP(R26) |
| pHDM280 | Trypsin | RVR/GLIP(R26) |
| pHDM281 | Trypsin | RR/GLIP(R26) |
| pHDM282 | Trypsin | EGGR/GLIP(R26) |
| pHDM284 | Trypsin | QAR/GLIP(R26) |
| pKVAM6 | Trypsin | QAR/V8-GLIP |
| pKVAM7 | Thrombin or Trypsin | LVPR/V8-GLIP |
| pHMM252 | Trypsin | QAR/V8-GLIP |
| pHMM253 | Thrombin or Trypsin | LVPR/V8-GLIP |

The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

Vectors suitable to carry the nucleic acids of the present invention comprise RNA viruses, DNA viruses, lytic bacteriophages, lysogenic bacteriophages, stable bacteriophages, plasmids, viroids, and the like. The most preferred vectors are plasmids.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably-linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extra-cellular export of a resulting polypeptide.

The present invention also provides a method for constructing a recombinant host cell capable of expressing BLT and other proteins, said method comprising transforming or otherwise introducing into a host cell a recombinant DNA vector that comprises an isolated DNA sequence that encodes the peptide of interest. The preferred host cell is any cell that can accommodate high level expression of an exogenously introduced gene or protein, such as *E.coli.* Transformed host cells may be cultured under conditions well known to skilled artisans such that recombinant protein is expressed.

The following examples more fully describe the present invention. Those skilled in the art will recognize that the particular reagents, equipment, and procedures described are merely illustrative and are not intended to limit the present invention in any manner.

### EXAMPLE 1

### Construction of a Synthetic Gene Encoding BLT

Plasmid constructions were performed using standard cloning methodologies as described by Maniatis et.al., Molecular Cloning: A laboratory Manual, Cold Spring Harbor Laboratory, N.Y. (1982). Enzymes and other reagents were obtained from Gibco-BRL, Gaithersgurg, MD or New England Biolabs, Beverly MA 01915. Methods for digesting, identifying, recovering and purifying the various oligonucleotides and DNA fragments used in this invention are known to those skilled in the art, as are methods for ligation of these sequences into vectors, transforming host microorganisms, and culturing these host microorganisms under conditions allowing production of plasmid DNA and recombinant protein products.

A DNA sequence encoding human BLT (SEQ ID NO:38 and SEQ ID NO:39) was assembled from 6 chemically synthesized oligonucleotides. Oligonucleotides were designed to construct a gene that would fuse the *b*-lipotropin coding sequence to the yeast alpha mating factor. The oligonucleotide sequences are shown below.

| Oligo Name | pM/u l | Oligo Sequence (5'>3') |
|---|---|---|
| Lars792 | 9.70 0 | tggaacactccctgctggttgctgcagagaagaaagac gaaggtccgtaccgtatggaacacttccgttggggttc cccgccgaaagacaag |
| Lars793 | 11.0 2 | gtaacgcttgtctttcggcggggaaccccaacggaagt gttccatacggtacggaccttcgtctttcttctctgca gcaaccagcagggagt |
| Lars794 | 8.48 5 | cgttacggtggtttcatgacctccgagaaatcccagac cccgctggttactctgttcaagaacgctatcatcaaga acgcgtacaagaaaggtgaataatag |
| Lars795 | 8.35 7 | gatcctattattcacctttcttgtacgcgttcttgatg atagcgttcttgaacagagtaaccagcggggtctggga tttctcggaggtcatgaaaccac |
| Lars796 | 4.68 7 | tcgagaaaagagaactgaccggtcagcgtctgcgcgaa ggtgacggtccggacggtccggctgacgacggtgctgg tgctcaggcagatc |
| Lars797 | 3.49 3 | gttccagatctgcctgagcaccagcaccgtcgtcagcc ggaccgtccggaccgtcaccttcgcgcagacgctgacc ggtcagttctcttttc |

25 ul of Lars792 and 25 ul of Lars793 were heated at 70°C for 15 minutes. These were combined and heated an additional 15 minutes at 70°C. The mixture was placed in a beaker of water at 50°C and removed to the benchtop until it had cooled to room temperature. This mixture is linker B. 25ul of Lars794 and 25ul of Lars795 were combined using the previous protocol. This mixture is linker C. 40ul of Lars796 and 50ul of Lars797 were combined using the previous protocol. This mixture is called Linker XhoI. Each linker mixture was kinased in a reaction set up as follows: 25ul of linker; 3 ul of 10X ligase buffer (Boehringer Mannheim); 1 ul of 10mM ATP; 1ul of T4 polynucleotide kinase (10U/ul; 3' phosphatase free; Boehringer Mannheim). The reaction was incubated for 30 minutes at 37°C; heat inactivated at 70°C for 15 minutes, and finally placed in a beaker of water at 50°C and removed to the benchtop until it had cooled to room temperature.

Ten (10) ug of pBCSKII(+) (Stratagene) was digested in a reaction as follows: 20ul of DNA; 3 ul of 10X H buffer (Boehinger Mannheim); 1ul of XhoI (10 units; Boehringer Mannheim); 1 ul of BamHI (10 units; Boehringer Mannheim) ; 5 ul of distilled water. This reaction was incubated at 37°C for 1 hour. The reaction was loaded on a small TBE 1% agarose gel and electrophoresed approximately 40 minutes at 140 volts. The gel was stained and the large approximately 2.9 kB band visualized on a UV lamp. A slit was made in front of the band and a piece of DEAE (Whatman) paper placed in the slit. The gel was electrophoresized an additional 10 minutes until all the DNA was collected on the DEAE paper. The paper was removed into a large Eppendorf microcentrifuge tube and incubated in 0.5 ml of 10mM tris-HCl pH 8.0, 1 M NaCl for 15 minutes. This solution was centrifuged at high speed for 5 minutes and the supernatant was removed to a new tube. The supernatant was mixed with 1 ml of 100% ethanol (-20°C) and the DNA pellet collected by centrifugation in a microfuge for 7 minutes. The pellet was resuspended in 100 ul of distilled water.

20 ul of the purified pBCSKII(+) (approximately 0.5ug) was mixed with 4 ul of the kinased linker B, 5 ul of the kinased linker C, and 11ul of the kinased linker XhoI. 5 ul of 3.0 M sodium acetate pH 8.0 was added and the solution mixed well. 100 ul of cold 100% ethanol was added. The solution was mixed well and centrifuged for 7-8 minutes at high speed in a microfuge. The pellet was resuspended in 10 ul of the ligation reaction mixture : 1ul of 10X ligase buffer (Boehringer Mannheim); 1ul of T4 ligase (10U; Boehringer Mannheim); and 8 ul of distilled water. This ligation was kept at room temperature for 1 hour and 5 ul used to transform *E.coli* K12 DH5a. The cells were plated on T-agar containing chloramphenicol (Sigma) (50 ug/ml) and X-gal (Boehringer Mannheim) (50ug/ml). Transformants that were white were grown in L-broth containing chloramphenicol (50ug/ml). One clone was identified to contain the desired sequence and was designated as pLGD700.

### EXAMPLE 2

### Recombinant Vector pHMM260-ProCpB(10)/LVPR/Beta lipotropin (R49) Encoding Procarboxypeptidase-BLT Fusion Protein

A recombinant DNA expression vector, pHMM260, contains a ProCpB(10)/LVPR/Beta Lipotropin (R49) expression cassette, encoding a procarboxypeptidase-BLT fusion protein (SEQ ID NO:5 and SEQ ID NO:6). This vector was produced using standard cloning techniques. The expression cassette is flanked by an NdeI site at the 5' end and a BamHI site at the 3' end. The pro-peptide portion of porcine Procarboxypeptidase B protein is positioned at the 5' end of the cassette, beginning with a Met residue and ending with a Ser residue. A thrombin protease recognition sequence, LVPR, occurs immediately 3' of the procarboxypeptidase sequence. The sequence beginning with a Glu residue to the 3' end of the thrombin cleavage site encodes wild-type human beta-lipotropin.

Oligonucleotide primers MM402 and MM403 were synthesized by and obtained from Genosys Biotechnologies. Oligonucleotide dry pellets were resuspended in water (50 mM) for use as PCR primers and then incubated for 15 minutes at 70°C. 1 ml of MM402 and 1 ml of MM403 were combined with 1 ng of plasmid pLGD700, PCR buffer (Perkin Elmer, hereinafter abbreviated PE), dNTPs (PE), and AmpliTaq DNA polymerase (PE) and PCR amplified on a GeneAmp PCR System 9600 (PE). The conditions used for PCR amplification were 94°C for 2 minutes, followed by 30 cycles of 94°C for 30 seconds, 58°C for 30 seconds, 72°C for 30 seconds, followed by 72°C for 10 minutes, followed by a 4°C hold overnight. 10 ml of the PCR reaction was run on a 1% agarose gel to verify amplification of the correct size fragment (0.328 kb).

The manufacturer's protocol for the Original TA Cloning Kit (Invitrogen) was followed by combining 1 ml of the PCR reaction with kit-supplied pCR2.1, ligase buffer, and ligase, and incubated at 15°C overnight. 2 ml of the ligation reaction was then transformed into *E.coli* K12 DH5a cells. Transformants were selected on L-agar containing 50 mg/ml kanamycin (Sigma) and 40 mg/ml X-gal (Sigma). White, kanamycin-resistant transformants containing the desired pHMM254 construct were screened and identified by restriction enzyme analysis. The DNA sequence of the XhoI/BamHI-flanked Beta Lipotropin PCR product (hereinafter referred to as b-LT(R49) gene) was confirmed by DNA sequence determination in the LRL Molecular Biology Core Laboratory.

3 mg of the sequence-confirmed plasmid pHMM254 and 3 mg of plasmid pKVAM6 were independently digested with 10 units each of restriction enzymes XhoI (BM) and BamHI (BM) in buffer B (BM) for 1 hour at 37°C. The 3.9 kb expression plasmid backbone of pKVAM6 and the 0.306 kb b-LT(R49) gene from plasmid pHMM254 were gel-purified using a 1% SeaPlaque GTG low-melting point (LMP) agarose gel (FMC BioProducts, hereinafter abbreviated FMC), run in 1X Tris Acetate Buffer (TAE), by cutting the bands of interest from the gel with a scalpel. These LMP fragments were then melted at 70°C for 10 minutes and stored at 45°C until used in the ligation reaction.

1 ml of the LMP fragment containing the plasmid backbone for pKVAM6 and 4 ml of the LMP fragment containing the b-LT(R49) gene from plasmid pHMM254 were combined with 10 units of T4 ligase (BM) and ligase buffer (BM) in a 10 ml reaction volume, incubated at 15°C overnight, then used to transform *E.coli* K12 DH5a cells. Transformants were then selected on L-agar containing 50 mg/ml tetracycline (Sigma). Tetracycline-resistant transformants containing the desired pHMM260 construct were screened and identified by restriction enzyme analysis.

| Oligonucleotide Sequence Table | |
|---|---|
| Oligo Name | Oligo Sequence (5'-> 3') |
| MM4 02 | GCGGCCGCCTCGAGGCTCAGTTTGACAGCCTGGTACCGCGT GAACTGACCGGTCAGCGTCTGCGCG |
| MM403 | GCGGCCGCGGATCCCTATTATTCACCTTTCTTGTACGCGTT CTTG |

Several derivative cassettes were constructed such that position 49 of the BLT sequence was changed from the wild type sequence Arg (R49) to Ala (A49) pHMM261; or to Glu (E49) pHMM262; or to Gln (Q49) pHMM263. These derivative were designed to reduce susceptibility to proteolysis during synthesis and purification of the fusion proteins. All of these vectors encode soluble fusion proteins.

### EXAMPLE 3

### Purification of procarboxypeptidase fusion proteins from E.coli

*E.coli* cells transformed with a vector encoding a procarboxypeptidase-BLT fusion protein are grown as described in Example 7, induced with 100 uM IPTG, and lysed in 8.0 M urea, 50 mM Tris, pH 6.0. The lysate was passed through a DEAE column in the presence of 8M urea and eluted with a salt gradient from 0- 1 M NaCl. Fractions containing the fusion protein were loaded onto a reverse phase (C-18) column and the column washed with 0.1% trifluroacetic acid (TFA). The fusion protein was eluted with a gradient of 0-50% acetonitrile (in 0.1% TFA). The purified fusion protein was diluted 1:50 with 50 mM ammonium acetate, 150 mM NaCl pH 6.0 and the protein concentration adjusted to 0.5 mg/ml using an Amicon stirred cell concentrator with a YM 10 membrane. The recovered fusion protein was digested with 20 units of bovine thrombin per mg of fusion protein. The digestion was allowed to proceed for 16 hours at 40° C. The digested protein was fractionated on a C-18 reverse phase column and fractions containing BLT were identified by analytical HPLC and mass spectrometry.

### EXAMPLE 4

### Construction of Alpha Mating Factor-Propeptide-BLT Fusion

A fusion construct comprising BLT fused to the alpha mating factor was prepared in order to express the fusion protein in *Pichia pastoris.* pLGD700 was cut with BamHI and XhoI and the 284 bp fragment containing the BLT gene isolated using DEAE paper. Plasmid pLGD660 was cut as above and the 4.294 kB band isolated by using DEAE paper. 20 ul of the isolated band from pLGD700 was mixed with 10 ul of the isolated band from pLGD660 and the DNA was co-precipitated. The co-precipitated DNAs were ligated and used to transform *E.coli* DH5a. An ampicillin-resistant clone was identified by restriction analysis as containing the BLT sequence fused to the complete alpha mating factor sequence. This plasmid is called pLGD707.

A fusion of the BLT sequence to the propeptide at an XhoI site near the carboxy terminus of the propeptide creates a hybrid at the C-terminal end of the propeptide that includes the kex2 protease cleavage site (LysArg) of the alpha mating factor. For this purpose, pLGD707 was cleaved with XhoI and XbaI which releases a 457 basepair fragment containing a tripeptide sequence from the alpha mating factor, Glu Lys Arg, fused to the N-terminus of BLT. The plasmid pLGD318 was also cut with XbaI and XhoI in reaction conditions and the 7.9 kB fragment gel purified. 10ul of the purified fragment from pLGD707 was precipitated with 20ul of the band isolated from pLGD318 and a 10ul ligation set up as previously described. The ligation was used to transform *E.coli* DH5a. The plasmid pLGD733 (Figure 2) was identified as having the expected restriction pattern. This plasmid contains the BLT fused to the propeptide and containing the signal peptide from human serum albumin.

To create the alpha mating factor propeptide fusion, pLGD733 was cleaved with SfuI and BamHI. A 533 bp restriction fragment was gel purified. pLGD320 was restricted with the enzymes SfuI and BamHI. An 8 Kilobase restriction fragment from pLGD320 was gel purified as described. 50ul of the pLGD733 fragment was mixed with 10ul of the fragment from pLGD320, and the DNA mixture precipitated with cold ethanol. The precipitate was collected and ligated with T4 DNA ligase (Boehringer Mannheim)). The reaction was used to transform *E.coli* DH5a. Ampicillin resistant transformants were grown in L-broth with ampicillin (50ug/ml), miniprepped, and restriction analyzed. The plasmid pLGD745 (Figure 3) was identified as containing the desired contiguous alpha mating factor, propeptide, kex2 site, BLT fusion downstream from the *Pichia pastoris* promoter for the alcohol oxidase I gene.

Finally, the expression cassette from pLGD745 was subcloned into the plasmid pLGD587. pLGD587 has a pGAP-GFP expression cassette that has been cloned between the HIS4 and the 3'AOX1 of the *Pichia pastoris* plasmid. This cassette can be detected in *Pichia pastoris* by detection of green fluorescence when cells are illuminated with long wave UV light. pLGD745 was cleaved with NsiI and XbaI (Boehringer Mannheim). 25ul of pLGD587 was cleaved similarly only adding 3ul of 10X H buffer. Both reactions were incubated at 37°C for 1 hour and then run on a 1% agarose TBE gel. The 1.254 kB restriction fragment from pLGD745 and the 8.675 kB restriction fragment from pLGD587 were gel purified using the DEAE paper method previously described. 20 ul of the pLGD745 fragment was mixed with 10ul of the pLGD587 fragment and precipitated. The precipitate was collected by centrifugation, dried in a speed-vac, and ligated by addition of : 8ul water, 1ul 10X ligase buffer (Boehringer Mannheim), 1ul of T4 DNA ligase (Boehringer Mannheim). After at least one hour at room temperature, 5ul of this ligation is used to transform *E.coli* DH5a. The transformation is plated on L-agar with ampicillin (50ug/ml). Transformants resistant to ampicillin were picked into 10 ml of L-broth with ampicillin (50ug/ml), grown overnight at 37°C , and miniprepped. The plasmids were restriction mapped and the plasmid pLGD749 identified. This plasmid contains the pAOX1 driven amf-propeptide-kex2-*b*-lipotropin fusion cassette as shown previously in the case of pLGD745.

### EXAMPLE 5

### Transformation of pLGD749 into Pichiapastoris and detection of b-lipotropin:

The *E.coli* strain containing pLGD749 was grown in 100 ml of L-broth with ampicillin (50ug/ml) overnight at 37°C. The cells were collected by centrifugation and maxi-prepped according to the specifications of the QIAGEN maxiprep kit. Approximately 20ug of pLGD749 was cut with NotI (New England Biolabs) in a reaction as follows: 60ml pLGD749; 10ul of 10X Buffer (New England Biolabs), 2ul of NotI enzyme, 28 ul of water. The reaction was incubated at 37°C for 2 hours, heat inactivated at 70°C for 15 minutes, and precipitated with cold ethanol.

*Pichia pastoris* strains were prepared for transformatino according to the following protocol: DAY 1:
a.) A colony of *P. pastoris* recipient strain in 50mL YPD (General Laboratories, K202, supplied as 450ml bottle, supplemented with 50 ml of 20% dextrose (Sigma), 1ml of biotin (Sigma; 1mg/ml))
b.) 30°C x 48hrs, 275RPM

### DAY 3:

a.) Subculture 10µl, 30µl and 10µl culture into 100mL YPD
b.) 30°C x 16hrs (o/n), 275rpm

### DAY 4:

a) OD600 all cultures until one is between OD600 0.8-1.0)
b) Pellet culture by 5 min centrifugation
c) Rinse pellet 2x with cold sterile water with intermittent centrifugation
d) Rinse with cold sterile 1M Sorbitol (Sigma).
e) Resuspend pellet in 1M sorbitol (600µl per 100mL culture)
f) Dispense 50µl cells to spin tubes (2.0 ml Eppendorf) containing linearized plasmid pLGD749 DNA (10µl)
g) Ice x 25 min.
h) Transfer to bottom of cold electroporation cuvette with 0.2 cm electrode gap (BioRad)
i) Electroporate (BioRad Gene Pulser) 25µFD 200ohms 2.0kV (time constants should be near 4.7)
j) Add 0.5mL YPD media to cuvette then transfer solution to capped tube
k) Gently shake 30°C x 30 min
l) Plate cells (20µl, 100µl) onto agar plates containing minimal dextrose agar (MD) (General Laboratories, K-1001, 450 ml/bottle, supplement with 50 ml 20% dextrose, 1ml biotin (2mg/ml))
m) Incubate 2-4 days at 30°C.

Strains used were GS115 (*his4*) and SMD1163 (*his4, pep4*), available from Invitrogen. Transformants on MD plates were screened for GFP production by viewing under a long wave UV transilluminator. Fluorescent colonies were picked and single colonies streak purifed on MD plates. These were grown under conditions to induce the alcohol oxidase promoter. The fermentation protocol was as follows:
1.) Colony is picked into a 250 ml flask containing 100 ml of buffered minimal glycerol-complex medium (BMGY; General Laboratories K-563, 450ml/bottle , supplemented with 20 ml of 50% glycerol (Sigma) and lml biotin (2mg/ml)), and grown 2 days at 30°C shaken at 250 rpm.
2.) Culture is spun down in a 250 ml centrifuge bottle (Nalgene) using a GSA rotor in a Sorval RC-5B centrifuge.
3.) Culture is resuspended in buffered minimal methanol-complex medium (BMGY; General Laboratories K-563, 450ml/bottle , supplemented with 2 ml of methanol (Mallincroft) and lml biotin (2mg/ml)), and grown 2 days at 30°C shaken at 250 rpm.
4.) Culture is spun down in a 50 ml centrifuge tube (Falcon) using a Beckman Table top centrifuge.
5.) Supernatant containing secreted proteins is collected and stored at 4°C.

The supernatants from colonies were examined on 4-20% gradient SDS PAGE gels (Novex) by mixing 100 ul with 100 ul of 2X loading dye (Novex), heating at 37°C for 15 minutes and loading 40 ul on a 10 well gel. Gels are either stained using Gelcode (Pierce) according to the manufactures instructions or blotted on nitrocellulose and examined by Western analysis using antibody to β-endorphin (Peninsula Laboratories). Transformants were identified that produced b-lipotropin as a fusion with the propeptide and processed at the kex2 site based on size of the molecules.

### EXAMPLE 6

### Construction of pHMM69 Encoding PCpB/RVR/hPTH

Oligonucleotides MM105, MM106, MM107, and MM108 were synthesized. Oligonucleotides MM106 and 107 were phosphorylated, and complementary oligonucleotides were annealed as two independent modules (MM105/MM106, MM107/MM108), and ligated together to create an XhoI/BamHI-flanked synthetic gene endcoding ProCpB(10) C-term/RVR/human parathyroid hormone (1-34) (hereinafter abbreviated RVR/hPTH(1-34)). Purified oligonucleotide dry pellets were resuspended in water (1 ug/ul) and incubated for 15 minutes at 70°C. 8 ul of oligonucleotides MM106 and MM107 were independently phosphorylated in 20 ul reactions containing 1mM ATP (pH 8), 20 units of T4 polynucleotide kinase (New England Biolabs, hereinafter abbreviated NEB) and ligase buffer (NEB) for 15 minutes at 37°C. 1 ul of 1mM ATP and 10 units of kinase were added and the reaction was incubated for a further 15 minutes at 37°C. 8 ug of complementary oligonucleotides MM105/MM106 and MM107/MM108 were combined, incubated for 10 minutes at 70°C, and then cooled for 1 hour from 60°C to room temperature (25°C). The annealed modules were then combined and ligated in a 115 ul reaction volume by adding ligase buffer (NEB), 1 mM ATP, and 110 units of T4 ligase (NEB). The reaction was incubated at room temperature (25°C) for 1 hour followed by an overnight incubation at 15°C.

5 ug of plasmid pBluescript SK(-) (Stratagene) (GenBank# X52324) was digested with 10 units each of restriction enzymes XhoI (Boehringer-Mannheim, hereinafter abbreviated BM) and BamHI (BM) in buffer B (BM) for 1 hour at 37° C. The 2.9 kb plasmid backbone was gel-purified using a 1% SeaPlaque GTG low-melting point (LMP) agarose gel (FMC BioProducts, hereinafter abbreviated FMC), run in 1X Tris Acetate Buffer (TAE), by cutting the band of interest from the gel with a scalpel. The LMP fragment was then melted at 70°C for 10 minutes and stored at 45°C until used in the ligation reaction.

Approximately 0.15 ug of gel-purified plasmid pBluescript SK (-) and 0.5 ug of the RVR/hPTH(1-34) synthetic gene ligation were combined with 10 units of T4 ligase (BM) and ligase buffer (BM) in a 10 ul reaction volume, incubated at 15°C overnight, then used to transform *E.coli* K12 DH5α cells. Transformants were selected on L-agar containing 50 ug/ml ampicillin (Sigma) and 40 ug/ml X-gal (Sigma). White, ampicillin-resistant transformants containing the desired pHMM62 construct were screened and identified by restriction enzyme analysis. The DNA sequence of the XhoI/BamHI-flanked hPTH(1-34) synthetic gene insert was confirmed by DNA sequence determination in the LRL Molecular Biology Core Laboratory.

Oligonucleotides MM139 and MM140 were synthesized and purified using an OPC cartridge purified per manufacturer's protocol (ABI). Oligonucleotides MM139 and 140 were annealed to create an XhoI/NsiI-flanked synthetic adaptor endcoding the factor Xa protease recognition sequence, IEGR (hereinafter abbreviated Xa). OPC cartridge purified oligonucleotide dry pellets were resuspended in water (100 pmol/ul) then incubated for 15 minutes at 70°C. The Xa adaptor was created by combining 100 pmol of complementary oligonucleotides MM139/MM140 followed by a 10 minute incubation at 70°C, then cooled for 1 hour from 60°C to room temperature (25° C) .

5 ug of the sequence-confirmed plasmid pHMM62 was digested with 10 units each of restriction enzymes XhoI (BM) and NsiI (BM) in buffer H (BM) for 1 hour at 37°C. The 3.0 kb expression plasmid backbone of pHMM62 was gel-purified using a 1% SeaPlaque GTG low-melting point (LMP) agarose gel (FMC), run in 1X Tris Acetate Buffer (TAE), by cutting the band of interest from the gel with a scalpel. The LMP fragment was then melted at 70°C for 10 minutes and stored at 45°C until used in the ligation reaction.

Approximately 0.18 ug of gel-purified plasmid fragment from pHMM62 and 100 pmol of the Xa adaptor were combined with 2000 units of T4 ligase (NEB) and ligase buffer (NEB) in a 10 ul reaction volume, incubated at 15°C overnight, then used to transform *E.coli* K12 DH5a cells. Transformants were selected on L-agar containing 50 ug/ml ampicillin (Sigma) and 40 ug/ml X-gal (Sigma). White, ampicillin-resistant transformants containing the desired pHMM65 construct were screened and identified by restriction enzyme analysis. The DNA sequence of the XhoI/BamHI-flanked Xa/hPTH(1-34) synthetic gene insert was confirmed by DNA sequence determination.

Approximately 9 ug of the sequence-confirmed plasmid pHMM65 and 5 ug of plasmid pLGD150 were independently digested with 10 units each of restriction enzymes XhoI (BM) and BamHI (BM) in buffer B (BM) for 1 hour at 37°C. The 6.3 kb expression plasmid backbone of pLGD150 and the 0.141 kb Xa/hPTH(1-34) synthetic gene fragment from plasmid pHMM65 were gel-purified using a 1% SeaPlaque GTG low-melting point (LMP) agarose gel (FMC), run in 1X Tris Acetate Buffer (TAE), by cutting the bands of interest from the gel with a scalpel. These LMP fragments were then melted at 70°C for 10 minutes and stored at 45°C until used in the ligation reaction.

2 ul of the gel-purified plasmid backbone LMP fragment from pLGD150 and 2 ul of the gel-purified Xa/hPTH(1-34) gene fragment from pHMM65 were combined with 10 units of T4 ligase (BM) and ligase buffer (BM) in a 10 ul reaction volume, incubated at 15°C overnight, then used to transform *E.coli* K12 DH5a cells. Transformants were then selected on L-agar containing 50 ug/ml tetracycline (Sigma).

Tetracycline-resistant transformants containing the desired pHMM69 construct were screened and identified by restriction enzyme analysis. Approximately 100 ng of plasmid DNA from pHMM69 was transformed into *E.coli* K12 RV308 cells. Transformants were selected on L-agar containing 50 ug/ml tetracycline (Sigma). Tetracycline-resistant transformants containing the desired pHMM69 construct were screened and identified by restriction enzyme analysis. Phenotype plating analysis confirmed that the host strain was RV308, as it possessed a lac⁻ and Str^{r} phenotype.

| Oligonucleotide Sequence Table | |
|---|---|
| Oligo Name | Oligo Sequence (5'-> 3') |
| MM105 | TCGAGGCTCAGTTTGACAGCAGAGTCCGTTCTGTTTCTGAA ATCCAGCTGATGCATAACCTGGGCAAACATCTGAACT |
| MM106 | ATGTTTGCCCAGGTTATGCATCAGCTGGATTTCAGAAACAG AACGGACTCTGCTGTCAAACTGAGCC |
| MM107 | CTATGGAGCGTGTAGAATGGCTGCGTAAGAAGCTGCAGGAT GTTCACAACTTTTGATAGG |
| MM108 | GATCCCTATCAAAAGTTGTGAACATCCTGCAGCTTCTTACG CAGCCATTCTACACGCTCCATAGAGTTCAG |
| MM139 | TCGAGGCTCAGTTTGACTCGATCGAAGGCCGTTCTGTTTCT GAAATCCAGCTGATGCA |
| MM140 | TCAGCTGGATTTCAGAAACAGAACGGCCTTCGATCGAGTCA AACTGAGCC |

### EXAMPLE 7

### Expression, heat extraction, and Factor Xa protease processing of ProCpB(10)/Xa/hPTH(1-34) fusion protein from pHMM69/RV308

A single colony of the expression host strain for ProCpB(10)/Xa/hPTH(1-34), pHMM69/RV308, was grown up in 10 ml of L broth containing 12.5 ug/ml tetracycline (Sigma) at 30 °C overnight. This culture was diluted 1:25 into the 100 ml of L broth containing 12.5 ug/ml tetracycline (Sigma) and grown at 30 °C to an OD₅₅₀ of approximately 1.0, at which point the culture was induced by shifting the temperature to 37 °C. For Coomassie-stained SDS-PAGE analysis, one 10 ml and three 5 ml samples were collected at 0, 2, 4, and 6 hours post-induction, noting the OD₅₅₀ at each timepoint, into 15 ml Falcon tubes (Becton Dickinson, hereinafter abbreviated as BD). The cell pellets of the samples were prepared by centrifugation in a tabletop centrifuge (Jouan CR 4 22) at 4000 RPM, 10 minutes, 4 °C. The 10 ml samples, which were destined for HPLC purification, were prepared by adding 10 ml of 50% methanol to the centrifuged pellets followed by storage at 4 °C.

The promoter contained on the pHMM69 plasmid is P₅₄₈, a derivative of the lambda phage P_{L} promoter. This promoter system is controlled by the plasmid-encoded, temperature-sensitive repressor protein cI₈₅₇, which at 30 °C remains bound to the O_{L} operator within the P₅₄₈ promoter, preventing transcription initiation. At 37 °C, however, the cI₈₅₇ repressor protein undergoes a conformational change that causes it to dissociate from the operator, thus allowing RNA polymerase to bind to the promoter and initiate transcription.

To confirm the induced expression of the 15.4 kDa ProCpB(10)/Xa/hPTH(1-34) fusion protein, the samples were run on a 16% Tricine SDS-PAGE gel (NOVEX) per manufacturer's protocol. The loading of the samples was standardized by resuspending the 5 ul cell pellets in 200 ul/OD₅₅₀ of SDS-PAGE loading buffer containing b-mercaptoethanol, then loading 20 ul samples per well. The protein bands were visualized by Coomassie staining. The major band in each of the timepoints examined, except for the uninduced control, corresponds to the 15 kDa ProCpB(10)/Xa/hPTH(1-34) fusion protein.

Heat extraction of the fusion protein was carried out as follows. 5 ul of the 6 hour post-induction sample cell pellet was resuspended in 5 ml of TE/Sulfotolysis buffer (50 mM Tris [pH 8.5], 5 mM EDTA, 5 mM K₂S₄O₆, 100 mM Na₂SO₃). 3 ml of this suspension was transferred to a 5 ml borosilicate tube and heated in a 70 °C water bath for 6 minutes before being transferred to an ice bath. The sample was transferred to a 15 ml Falcon tube (BD) and centrifuged in a tabletop centrifuge (Jouan CR 4 22) at 4000 RPM, 10 minutes, 4 °C. The supernatant was removed to a fresh 15 ml Falcon tube (BD). 100 ul of the heat-extracted supernatant was combined with 100 ul of 2X SDS-PAGE loading buffer containing b-mercaptoethanol and boiled for 10 minutes. 20 ul of the sample was then loaded to a 16% Tricine SDS-PAGE gel (NOVEX) and run per manufacturer's protocol. The protein bands were visualized by Coomassie staining. Approximately 90% of the protein in the heat-extracted supernatant was the ProCpB(10)/Xa/hPTH(1-34) fusion protein, with about 10% *E.coli* background proteins.

700 ug of ProCpB(10)/Xa/hPTH(1-34) fusion protein was HPLC-purified and quantitated by the LRL Bioanalytical Development Lab. The 10 ul, 50% methanol pellet of the 4 hour timepoint was centrifuged for 10 minutes at 3000 RPM in a Beckman TJ6 centrifuge with a bucket rotor. The supernatant was decanted and discarded. 2 ul of 7M Guanidine HCl/50mM Tris (pH 8.5) was added to the tube along with a stir bar and the mixture was stirred vigorously for 1 hour. The stir bar was removed and the tube was centrifuged for 20 minutes at 3000 RPM in a Beckman TJ6 centrifuge with a bucket rotor. The supernatant was then filtered through a 0.45 micron filter into an HPLC injector vial. The HPLC unit used was comprised of an HP1090L with DR5-SDS, autosampler (10x10+1), autoinjector (250 ml), column oven, Waters 441 detector @ 214 nm with a Zinc lamp, attached to an HP1000 data system. Six 250 ul injections were loaded onto a semipreparative column (Vydac C4) at 0% mobile phase B (0.1% trifluoroacetic acid in 90% acetonitrile, 10% deionized water), 1 ul/min, 60 °C at 2 minute intervals. The system was bumped to 25% mobile phase B and the column was equilibrated for 10 minutes at 3 ul/min. A 120 minute gradient was run from 25-55% mobile phase B to mobile phase A (0.1% trifluoroacetic acid in 10% acetonitrile, 90% deionized water) and fractions were collected at 1 minute intervals during the gradient elution. Fractions 76-78 were pooled, mixed and split into two tubes, which were Speed-Vac'd to dryness. Both tubes contained approximately 700 ug of ProCpB(10)/Xa/hPTH(l-34) fusion protein. One of these tube was submitted for N-terminal amino acid sequencing and electrospray mass spectrometry, which confirmed the identity of the HPLC-purified material as of ProCpB(10)/Xa/hPTH(1-34) fusion protein.

The second tube of lyophilized fusion protein was reconstituted in 700 ul of Factor Xa cleavage buffer (20 mM Tris [pH 7.4], 200 mM NaCl, 1 mM EDTA). A 1:200 ratio (w/w) of Factor Xa to fusion protein was determined to give the best conversion of ProCpB(10)/Xa/hPTH(l-34) to ProCpB(10)/Xa leader, by Coomassie stained SDS-PAGE gel analysis. 20 ug of ProCpB(10)/Xa/hPTH(1-34) fusion protein was combined with 0.1 ug of Factor Xa (New England Biolabs, hereinafter abbreviated NEB) in a 21 ul final reaction volume and incubated 19 hours at room temperature (25 °C). 20 ul of 2X SDS-PAGE loading buffer containing b-mercaptoethanol was added to the Factor Xa cleavage reaction and boiled for 10 minutes. 20 ul of this sample was then loaded to a 16% Tricine SDS-PAGE gel (NOVEX) and run per manufacturer's protocol. The protein bands were visualized by Coomassie staining. Video densitometry of the stained gel was employed to estimate the percentage of the fusion protein converted to the ProCpB(10)/Xa leader was approximately 85%, using the IS-1000 Digital Imaging System (Alpha Innotech Corporation).

To examine the specificity of the Factor Xa cleavage, LC-MS analysis was performed by the LRL Bioanalytical Development Lab. 300 ug of ProCpB(10)/Xa/hPTH(1-34) fusion protein was combined with 1.5 ug of Factor Xa (NEB) in a 300 ul final reaction volume and incubated 19 hours at room temperature (25 °C). This digest was run on LC-MS to separate the generated peptides and assign their mass and relative abundance. The results from this analysis were that 100% of the fusion protein was digested at the Factor Xa cleavage site, but further proteolytic processing also occurred. Approximately 80% of the ProCpB(10)/Xa leader was processed correctly into a peptide corresponding to amino acids 1-97 of the fusion protein, while about 20% of the fusion leader was further processed to a peptide corresponding to amino acids 1-84 of the fusion protein. Approximately 40% of the hPTH(1-34) peptide was processed correctly with Factor Xa, resulting in a peptide which corresponds to amino acids 98-131 of the fusion protein. Three other peptides were also detected at the following percentages: 111-131 (35%), 98-110 (15%), and 118-131 (10%).

## Claims

1. A fusion protein comprising a propeptide sequence of procarboxypeptidase B and an heterologous protein.

2. A fusion protein as in claim 1 wherein said heterologous protein is beta-lipotropin, hPTH, or GLIP.

3. A fusion protein comprising beta-lipotropin as in claim 2 having an amino acid sequence identified herein as SEQ ID NO:4 or SEQ ID NO:6.

4. An isolated nucleic acid compound encoding a protein of Claim 1, claim 2 or claim 3.

5. A vector comprising an isolated nucleic acid compound of Claim 4.

6. A host cell containing a vector of Claim 5.

7. A method for constructing a recombinant host cell having the potential to express a protein, said method comprising introducing into said host cell by any suitable means a vector of Claim 5.

8. A method for expressing a fusion protein in a recombinant host cell of Claim 6, said method comprising culturing said recombinant host cell under conditions suitable for gene expression.

9. A process for preparing a protein of interest comprising:
a. transforming a host with an expression vector that encodes a procarboxypeptidase fusion protein comprising said protein of interest;
b. culturing said transformed host under conditions that enable expression of said fusion protein;
c. purifying from said transformed host said fusion protein by any suitable means;
d. releasing said protein of interest from said fusion protein by any suitable chemical or enzymatic cleavage.

10. A protein produced by the method of Claim 9.
